# EUROPEAN PATENT SPECIFICATION

(11) **EP 4 142 860 B1**
(45) Date of publication and mention of the grant of the patent: **26.08.2026**
(21) Application number: 21799683.4
(22) Date of filing: 03.05.2021
(51) Int. Cl.: G06F 3/01, A61N 1/04, A61N 1/00, A61N 1/02, A61M 21/00, G16H 40/20, H04R 1/02, H04R 9/06, H04R 17/00, B33Y 80/00

(54) **COMMUNICATION DEVICES, METHODS, AND SYSTEMS**
KOMMUNIKATIONSVORRICHTUNGEN, -VERFAHREN UND -SYSTEME
DISPOSITIFS, PROCÉDÉS ET SYSTÈMES DE COMMUNICATION

(30) Priority: 02.05.2020 US 202063019302 P
(43) Date of publication of application: 08.03.2023
(73) Proprietor: DataFeel Inc., Washington, DC 20006 (US)
(72) Inventor: SEMBALUK, Leigh, Vancouver, BC V5L 4Y1 (CA); LEAPER, Matthew Robert, Washington, DC 20006 (US)
(74) Representative: HGF
(86) International application number: PCT/US2021/030528
(87) International publication number: WO 2021/225994

(56) References cited:
- EP-A1- 4 009 145
- WO-A1-2016/019250
- CA-A1- 3 092 689
- US-A1- 2006 052 695
- US-A1- 2015 335 288
- US-A1- 2017 080 255
- US-A1- 2019 151 604
- US-A1- 2019 278 374
- US-B1- 6 547 788
- SINGHAL ANSHUL ET AL: "Perceptual interactions in thermo-tactile displays", 2017 IEEE WORLD HAPTICS CONFERENCE (WHC), IEEE, 6 June 2017 (2017-06-06), pages 90 - 95, XP033131395, DOI: 10.1109/WHC.2017.7989882

## Description

### TECHNICAL FIELD

Aspects of the present disclosure generally relate to communication devices, methods, and systems. Particular aspects relate to wearable and implantable communication devices that are positionable adjacent physiologic tissue (e.g., skin) and communicable with the brain using nerves associated with the physiologic tissue.

### BACKGROUND

Computer screens have emerged as the most common means for person-to-computer communication. In 2015, for example, it was estimated that the average adult spends roughly 10 hours a day looking at a screen to consume information and/or communicate with others. The human eye was not designed for all this screen time, and numerous symptoms have been associated therewith. For example, eyestrain from hours of screen time may cause instances of eye irritation, dryness, fatigue, and/or blurred vision that last for extended periods of time. These problems are increasingly common, and the near constant production of new screen-oriented devices (e.g., the next iPhone^{®}) suggests further increases.

Alternate means for person-to-computer communications may reduce the negative effects of excessive screen time. For example, the human body includes many non-optical nerves that are capable of communicating data to the brain. The skin is the largest organ in the human body and serves multiple functions including those related to temperature modulation, immuno-regulation and sensory inputs. There is a vast network of nerves highly attuned to receiving environmental data and relaying them more centrally to the brain. It is this role of the peripheral nervous system which relays environmental inputs such as the nerves associated with the skin. Further improvements are required to better leverage these and other communication capabilities of our sensory organs. A conference paper from Anshul Singhal and Lynette A. Jones entitled "Perceptual interactions in thermos-tactile displays", 2017 IFEE WORLD HAPTICS CONFERENCE (WHC), IEEE, 6 June 2017, pages 90-95, XP033131395 describes an experiment that measured thermal pattern identification in the presence of concurrent vibrotactile feedback on the thenar eminence of the hand. In particular, a thermo-tactile display configured to simultaneously output thermal and tactile cues to the skin of a user is disclosed. The thermo-tactile display comprises an annular thermoelectric (Peltier) module with a central hole in which a vibration motor is arranged, such that there is a gap between the thermoelectric module and the vibration motor. The thermoelectric module together with the vibration motor is mounted on a heat sink, and a fan is provided for forced convection cooling. Patent document WO 2016/019250 A1 describes a system including patches and corresponding modules for wirelessly monitoring physiologic and/or physical signals.

### SUMMARY

Numerous aspects are disclosed in this application. One exemplary aspect is a communication device. For example, the device may comprise an apparatus comprising an energy generator comprising a plurality of generator elements operable to output a plurality of different energy types in a signal direction toward a physiologic tissue and a printed circuit board that mechanically supports and electrically connects the plurality of generator elements to each other. Each generator element of the plurality of generator elements may be independently operable, when the energy generator is positioned relative to the physiologic tissue, to communicate with different nerves associated with the physiologic tissue by outputting a different portion of an energy signal in the signal direction toward the physiologic tissue with one energy type of the plurality of different energy types.

The plurality of generator elements may comprise a vibratory generator element and a thermal generator element. The plurality of different energy types may comprise a vibratory energy output with the vibratory generator element and a thermal energy output with the thermal generator element. The vibratory generator element may comprise a coin vibration motor. The thermal generator element may comprise a thermoelectric generator operable with the Seebeck effect to create a temperature differential responsive to an electric current supplied to the thermoelectric generator. The thermoelectric generator may be operable responsive to the electric current to output a hot thermal energy and a cold thermal energy. The thermoelectric generator may comprise an open shape with a central opening and the vibratory generator is located in the central opening. The open shape of the thermoelectric generator may comprise an annular shape with a circular central opening and the vibratory generator comprises a circular shape located in the circular central opening to define a continuous air gap and thermal break between exterior surfaces of the vibratory generator and interior surfaces of the thermoelectric generator.

The plurality of generator elements may comprise an electrical stimulus generator element. The plurality of different energy types may comprise an electrical stimulus output with the electrical stimulus generator element. The plurality of different energy types may comprise an electrical stimulus output with the electrical stimulus generator element. The electrical stimulus generator element may comprise a pair of contact plates operable to output the electrical stimulus. The pair of contact plates may be structurally connected to the printed circuit board by an insulating material and electrical connected to the printed circuit board by a conductor. The pair of contact plates may be mounted directly to pads of the printed circuit board. The plurality of generator elements may comprise an electrical stimulus generator element comprising a pair of semi-annular contact plates that are positioned in the continuous air gap and to maintain the thermal break.

The plurality of generator elements may comprise a pressure generator element. The plurality of different energy types may comprise a pressure energy output with the pressure generator element. The pressure generator element may comprise a piezoelectric speaker operable to output the pressure energy responsive to an electric current supplied to the pressure generator element. A face of the piezoelectric speaker may be spaced apart from the physiologic tissue to define an air gap and the pressure energy comprises a sonic energy output toward the physiologic tissue through the air gap with the speaker.

The pressure generator element may comprise an array of piezoelectric speakers operable to output the pressure energy responsive to an electric current supplied to the pressure generator element. The plurality of generator elements may comprise a pressure generator element comprising a radial array of piezoelectric speakers. The radial array of piezoelectric speakers may be coaxial with and surrounding the annular shape of the thermoelectric generator. In any of these examples, the pressure generator element may comprise one or more ultrasound transducers.

The plurality of generator elements may comprise an optical generator element. The plurality of different energy types may comprise an optical energy output with the optical generator element. The optical generator element may comprise a multi-color LED that is operable to output the optical energy and mechanically supported and electrically connected to the printed circuit board.

The apparatus may comprise a sensor that is operable to detect physiological signals and mechanically supported and electrically connected to the printed circuit board at a location among the plurality of energy generating elements. The sensor may be operable to detect physiological signals comprising measurements of electrical activity produced by a beating heart. The sensor may be operable to detect physiological signals comprising measurements of electrical activity produced by movements. The sensor may be operable to detect physiological signals comprising measurements of electrical activity produced by breathing.

The apparatus may comprise a graspable body surrounding the plurality of energy generating elements. The graspable body may comprise a cylindrical shape made from an insulating material operable to limit outputs of the plurality of different energy types in directions away from the physiologic tissue. The apparatus may comprise a wearable body engageable with the printed circuit board to maintain a position of the plurality of energy generator elements relative to the physiologic tissue when the wearable body is worn. The wearable body may be configured to absorb a portion of the plurality of energies when the energy signal is output. The wearable body may comprise a crystalline structure or a polymeric structure.

The apparatus may comprise a support body engageable with the printed circuit board to maintain a position of the plurality of energy generator elements relative to the physiologic tissue when the support body is resting on the tissue. The support body may comprise a crystalline structure or a polymeric structure. The plurality of energies may be oriented toward an interior portion of the support body so that the energy signal is output to the physiologic tissue through the support body. A portion of the plurality of energies may be transferred to the support body when the energy signal is output.

The support body may comprise a translucent polymeric structure and additives that are suspended in the translucent polymeric structure create a visual or functional feature that is responsive to the energy signal. The plurality of energy generators comprise an optical generator element comprising a multi-color LED operable to illuminate the translucent polymeric structure in a plurality of different colors. The additives may comprise flecks of light reactive materials suspended in the translucent polymeric structure. The flecks may comprise a thermally reactive material suspended in the translucent polymeric structure to thermally conductive pathways.

In accordance with the present invention, an apparatus is provided as set out in independent claim 1. Further embodiments are defined in the dependent claims.

### BRIEF DESCRIPTION OF THE DRAWINGS

The accompanying drawings are incorporated in and constitute a part of this specification. These drawings illustrate exemplary aspects of the present disclosure that, together with the written descriptions provided herein, serve to explain the principles of this disclosure.
FIG. 1A depicts an exemplary energy signal output onto a living tissue;
FIG. 1B depicts an exemplary communication device configured to output the energy signal of FIG. 1A;
FIG. 2A depicts a top-down view of the FIG. 1B device;
FIG. 2B depicts a bottom-up view of the FIG. 1B device;
FIG. 2C depicts a cross-section view of the FIG. 1B device taking along section line A-A of FIG. 2A;
FIG. 3A depicts a cross-section of an exemplary energy generator;
FIG. 3B depicts a bottom-up view of the FIG. 3A generator;
FIG. 4A depicts an impact energy output with the FIG. 3A generator;
FIG. 4B depicts a heat energy output with the FIG. 3A generator;
FIG. 4C depicts an electrical energy output with the FIG. 3A generator;
FIG. 4D depicts a pressure energy output with the FIG. 3A generator;
FIG. 5 depicts an exemplary processing unit;
FIG. 6 depicts a cross-section of an exemplary energy generator;
FIG. 7 depicts a bottom-up view of the FIG. 6 generator;
FIG. 8A depicts an impact energy output with the FIG. 6 generator;
FIG. 8B depicts a heat energy output with the FIG. 6 generator;
FIG. 8C depicts an electrical energy output with the FIG. 6 generator;
FIG. 8D depicts a pressure energy output with the FIG. 6 generator;
FIG. 9 depicts a cross-section of an exemplary energy generator;
FIG. 10 depicts a bottom-up view of the FIG. 9 generator;
FIG. 11 depicts a cross-section of an exemplary energy generator;
FIG. 12 depicts a bottom-up view of the FIG. 11 generator;
FIG. 13A depicts a side view of the FIG. 6 generator;
FIG. 13B depicts a side view of the FIG. 6 generator when embedded in a graspable body of a data communication device.
FIG. 14A depicts a side view of the FIG. 9 generator when embedded in a wearable body of a data communication device.
FIG. 14B depicts a back view of the FIG. 9 generator when embedded in the wearable body of FIG. 14A.
FIG. 15 depicts a side view of the FIG. 11 generator when embedded in a support body of a data communication device.

### DETAILED DESCRIPTION

Aspects of the present disclosure are now described with reference to exemplary communication devices, methods, and systems. Particular aspects reference a healthcare setting, wherein the described devices, methods, and systems may allow a single caregiver to monitor vital signals for a plurality of patients without using a screen, or at least with a reduced amount of screen time. Any references to a particular setting, such as healthcare; a particular user, such as a caregiver; particular data, such as vital signals; or particular amount of screen time, are provided for convenience and not intended to limit the present disclosure unless claimed. Accordingly, the aspects disclosed herein may be utilized for any analogous communication device, method, or system - healthcare-related or otherwise.

The terms "proximal" and "distal," and their respective initials "P" and "D," may be used to describe relative components and features. Proximal may refer to a position closer to a hand of user, whereas distal may refer to a position further away from said hand. With respect to a hand adjacent a living tissue, for example, proximal may refer to a position away from the tissue, whereas distal may refer to a position toward said tissue. As a further example, with respect to energy directed toward the living tissue, proximal may refer to energy directed away from the tissue and distal may refer to energy directed toward the tissue. Appending the initials P or D to a number may signify its proximal or distal location or direction. Unless claimed, these directional terms are provided for convenience and not intended to limit this disclosure.

Aspects of this disclosure may be described with reference to one or more axes. For example, an element may extend along an axis, be moved along said axis in first or second direction, and/or be rotated about said axis in a first or second direction. One axis may intersect another axis, resulting in a transverse and/or perpendicular relationship therebetween. For example, two or three perpendicular axes may intersect at an origin point to define a Cartesian coordinate system. The directional terms proximal and distal may be used with reference to any axis. One axis may be a longitudinal axis extending along a length of an element, such as a central longitudinal axis extending along the length and through a centroid of the element.

Terms such as "may," "can," and like variation, are intended to describe optional aspects of the presentdisclosure. Terms such as "comprises," "comprising," or like variation, are intended to describe a non-exclusive inclusion, such that a device, method, or system comprising a list of elements does not include only those elements but may include other elements not expressly listed or inherent thereto. The term "and/or" indicates a potential combination, such that a first and/or second element may likewise be described as a first element, a second element, or a combination of the first and second elements. These potential combinations are provided as examples. Numerous other combinations are inherent to this disclosure. Unless stated otherwise, the term "exemplary" is used in the sense of "example" rather than "ideal."

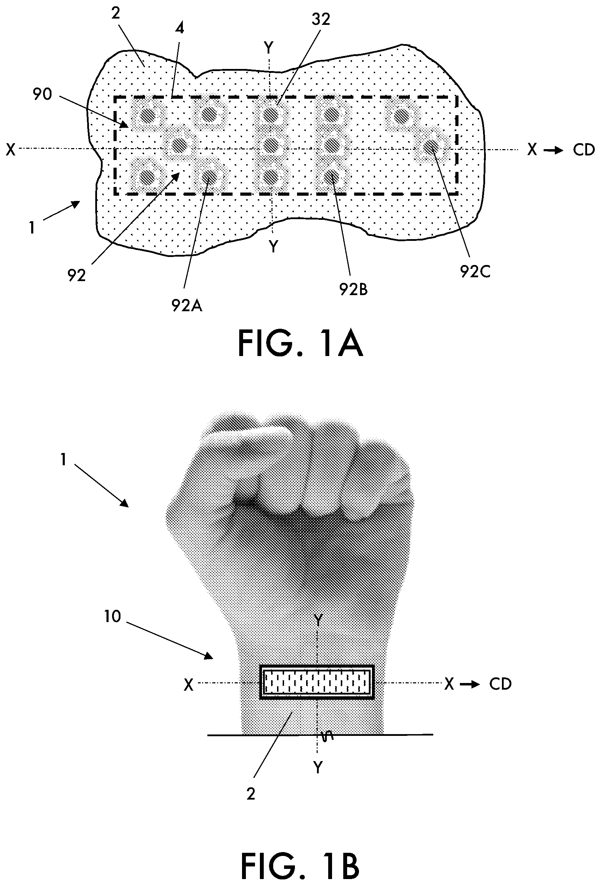

Aspects of this disclosure are directed to devices, methods, and systems for communicating with the brain through nerves associated with a living tissue. Some aspects are described with reference to an energy signal including one or more energies output to communicate symbols to the living tissue. The symbols may be used to communicate data, and the one or more energies may be used to communicate aspects of the data. The living tissue may be a portion of skin, as shown in FIGs. 1A-8D. In a healthcare setting, the energy signal may be output towards the skin of a caregiver to communicate symbols associated with a status of a patient. For example, an intensity of the one or more energies may escalate responsive to a measure of the status, providing a non-visual alert to the caregiver if the measure changes.

Exemplary energies and energy signals are now described with reference to FIG. 1A, which depicts an exemplary energy signal 90 including a plurality of symbols 92 output onto a communication area 4 of a physiologic tissue (e.g., skin 2) of user 1 with one or more different energies 32. For illustrative purposes, the symbols 92 of FIG. 1 are shown from a proximal-to-distal direction, as they would be output to the physiologic tissue (e.g., skin 2) by an energy transceiver. Each different energy 32 may communicate aspects of the data to the brain through nerves associated with the physiologic tissue (e.g., skin 2), such as nerves located distal of communication area 4.

The physiologic tissue may include skin 2 any underlying muscle, bone, and/or other portions of user 1 capable of receiving and responding to one or more different energies 32 during the second time period. For example, the one or more different energies 32 shown in FIG. 1A may be recognizable by nerves associated with skin 2 including: (i) touch receptors, such as the Meissner's corpuscle; (ii) temperature receptors, such the Ruffini corpuscle and Krause corpuscle; (iii) electrical receptors, such as the muscles and pain receptors located in the dermis layer; (iv) pressure receptors such as the Pacinian corpuscle; and/or (v) any other cutaneous or subcutaneous nerves that innervate skin 2 or other physiologic tissues and are responsive to energies 32.

Each symbol 92 may be associated with different data. For example, in the healthcare setting, each symbol 92 may be associated with a vital sign of the patient, such as body temperature, pulse rate, respiration rate, and/or blood pressure. As shown in FIG. 1A, the plurality of symbols 92 may include a first symbol 92A, a second symbol 92B, and a third symbol 92C. In keeping with the previous example, first symbol 92A may be associated with temperature and pulse rate, second symbol 92B may be associated with respiration rate, and third symbol 92C may be associated with blood pressure. Any number of symbols 92 may be provided and/or associated with a measurable or non-measurable characteristic of the patient.

Symbols 92A, 92B, and 92C are shown as pip patterns of dots in FIG. 1A, wherein each dot is a shaded area. Each dot may represent an output of the one or more different energies 32. Aspects of energies 32 and/or each symbol 92A, 92B, and 92C may increase the complexity of energy signal 90, and thus the amount of data transmitted therewith. As shown in FIG. 1A, symbols 92A, 92B, and 92C may be scrolled across communication area 4 by outputting energies 32 toward the skin in the pip patterns; and moving the patterns across the skin in a communication direction CD. In FIG. 1A, first symbol 92A is a pip five dot pattern; second symbol 92B is a pip six dot pattern; and a third symbol 92C is a pip three dot pattern that has been truncated by an end of communication area 4 due to the scrolling. Symbols 92 may be flashed and scrolled. For example, the five dots of first symbol 92A in FIG. 1A may be output to communicate a temperature range of the patient (e.g., a normal range), and flashed on-and-off to communicate the pulse rate of the patient.

An exemplary energy transceiver 10 is depicted in FIG. 1B as being configured output energy signal 90 to communication area 4 of a physiologic tissue o fuser 1 (e.g., skin 2). As shown, energy transceiver 10 may be attached to a portion of the physiologic tissue (e.g., skin 2), including any portion located on a limb, such as the underside of a human wrist shown in FIG. 1B for example. Communication area 4 may be sized approximate to a perimeter of transceiver 10. In this configuration, transceiver 10 may communicate energy signal 90 to the physiologic tissue (e.g., skin 2) by outputting the one or more different energies 32 toward communication area 4 in a signal direction oriented toward the physiologic tissue (e.g., skin 2). As shown in FIG. 1A, the energies 32 may be output individually and/or in combination to communicate aspects of any of symbols 92A, 92B, and 92C to the physiologic tissue (e.g., skin 2).

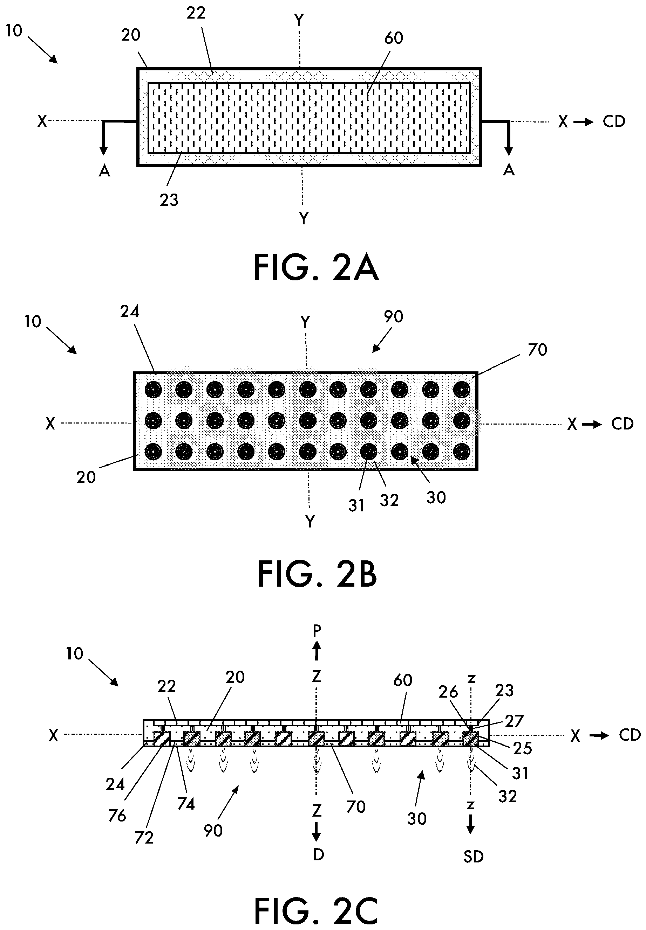

Additional aspects of exemplary energy transceiver 10 are now described with reference to FIGs. 2A-C. As shown, transceiver 10 may comprise: a body 20; a tissue interface 30; a processing unit 60; and an attachment element 70. With these elements, and the variations described herein, energy transceiver 10 may communicate energy signal 90 to nerves associated with the physiologic tissue (e.g., skin 2) by outputting the one or more different energies 32 towards the physiologic tissue with tissue interface 30.

As shown in FIGs. 2A-C, body 20 may contain the elements of energy transceiver 10. For example, body 20 of FIGs. 2A-C has a length extending along a longitudinal axis X-X, a width extending along a lateral axis Y-Y, and a thickness extending along a proximal-distal axis Z-Z. The length, width, and/or thickness of body 20 may be compatible with the physiologic tissue (e.g., skin 2). For example, body 20 may be composed of a flexible biocompatible base material, such as a polymeric material, so that the length and width of body 20 are conformable against a curvature of skin 2.

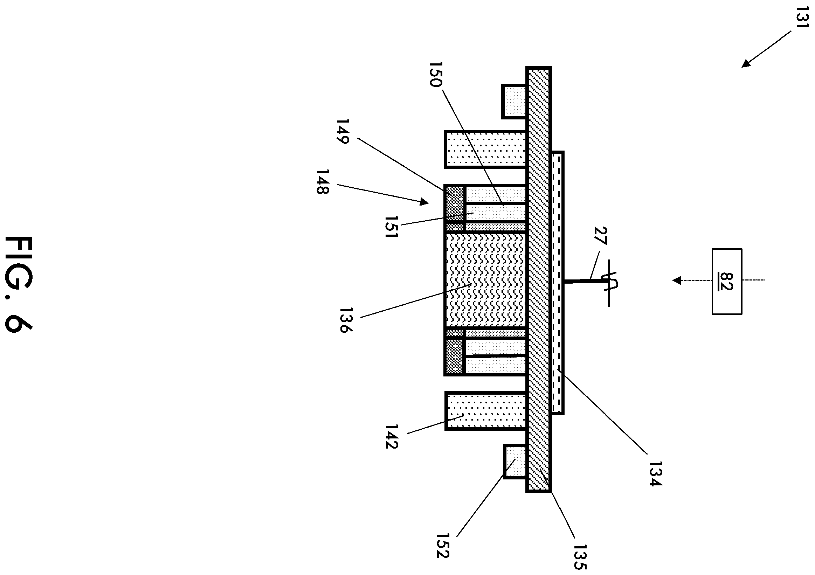

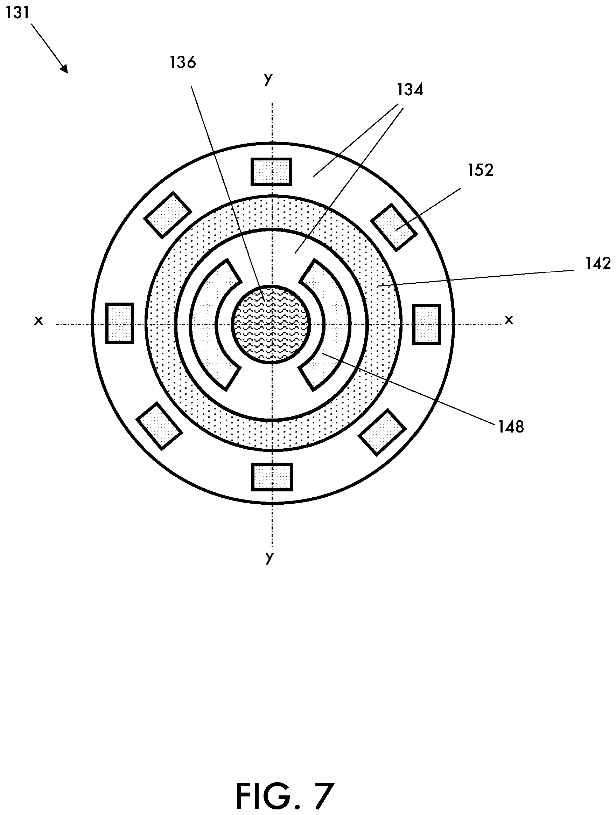

Body 20 may include any shape and be conformable with any curvature. For example, body 20 may be conformable with a cylindrical shape of a human forearm (e.g., FIG. 1B) and/or comprise a semi-spherical shape a human forehead or limb (e.g., FIGs. 6A, 6B, 6C, 6D), an irregular curved shape of a human foot (e.g., FIG. 7A), an irregular curved surface of a grip (e.g., FIG. 7B), and/or surfaces of an implant (e.g., FIGs. 7C, 7D). A plurality of bodies 20 may be joined together to accommodate some curvatures. For example, side surfaces of body 20 of FIGs. 2A-C may be removable engageable with side surfaces of additional bodies 20 to create a joined layer conformable with the curvature.

The base material of body 20 may have insulating and/or energy-directing properties. For example, the base material may include compositions and/or coatings that promote energy flows along proximal-distal axis Z-Z, and limit energy flows along axes X-X and/or Y-Y. Body 20 may be manufactured from the base material using any known process. For example, body 20 may be molded or 3D printed from a base material that is biocompatible, dielectric, impact resistance, sound absorbing, and/or thermally resistant, including any type of polymeric materials that are 3D printable, implantable (e.g., such as polyether ether ketone or PEEK), and have like characteristics. As a further example, body 20 may comprise any biocompatible metal (e.g., titanium) or metal alloy (e.g., stainless steel) implants or ceramic. Additional materials and/or coatings may be included with the base material and/or applied to body 20 to further promote biocompatibility.

As shown in FIGs. 2A-C, body 20 may define a proximal surface 22 (FIG. 2A) opposite of a distal surface 24 (FIG. 2B) along proximal-distal axis Z-Z (FIG. 2C). In FIGs. 2A and 2C, for example, proximal surface 22 includes a processor compartment 23 configured to receive processing unit 60. As shown, and described further below, processing unit 60 may be removable engageable (e.g., snap-fit into) with processor compartment 23. Body 20 may include and/or be compatible with additional mechanisms for securing and/or releasing the snap-fit, such as a retaining screw and/or a lever.

Body 20 of FIGs. 2A-C includes a plurality of communication bays 25. As shown, each communication bay 25 may be spaced apart from the next on distal surface 24 in a grid pattern. The spacing may be uniform or non-uniform. In FIGs. 2B and 2C, the bays 25 are spaced apart uniformly for communication with the physiologic tissue (e.g., skin 2) of FIG. 1B, which has a fairly planar surface area. Non-uniform spacing may be used to accommodate a curvature of the physiologic tissue (e.g., skin 2). As shown in FIG. 2C, each communication bay 25 may extend proximally into body 20 through distal surface 24 along a communication axis z-z that is parallel with the proximal-distal axis Z-Z of transceiver 10. In FIG. 2C, a conduit 26 extends proximally from each bay 25, through an interior portion of body 20, and into processor compartment 23, placing the plurality of bays 25 in communication with compartment 23.

Aspects of tissue interface 30 are now described with reference to FIGs. 2B and 2C. As shown, tissue interface 30 may include a plurality of energy generators 31, and each generator 31 may be located in one of communication bays 25. Each generator 31 may be operable with processing unit 60 to output energies 32 individually and/or in combination. In FIGs. 2B and 2C, for example, the one or more different energies 32 are being output from the shaded generators 31 to communicate energy signal 90 of FIG. 1A. As shown in FIG. 2C, one or more conductors 27 may extend through each conduit 26 to connect processing unit 60 to each energy generator 31, allowing control signals to be transmitted between processing unit 60 and the plurality of energy generators 31 along one or more pathways.

As shown in FIG. 2C, the one or more conductors 27 may include any number of electrical wires and/or optical fibers configured to transmit the control signals. For example, the conductors 27 may comprise a plurality of electrical conductors interconnecting the plurality of generators 31 with processing unit 60, and allowing electricity-based control signals, energies, and communications to be transmitted between unit 60 and generators 31. In addition, or alternatively, the conductors 27 may comprise a plurality of optical fibers interconnecting the plurality of generators with processing unit 60, and allowing light-based control signals, energies, and communications to be transmitted between unit 60 and generators 31. For example, each conductor 27 may comprise a twisted pair including at least one electrical conductor and at least one optical fiber. A flexible energy-insulating medium, such as an epoxy, may be used to seal conductors 27 in conduits 26.

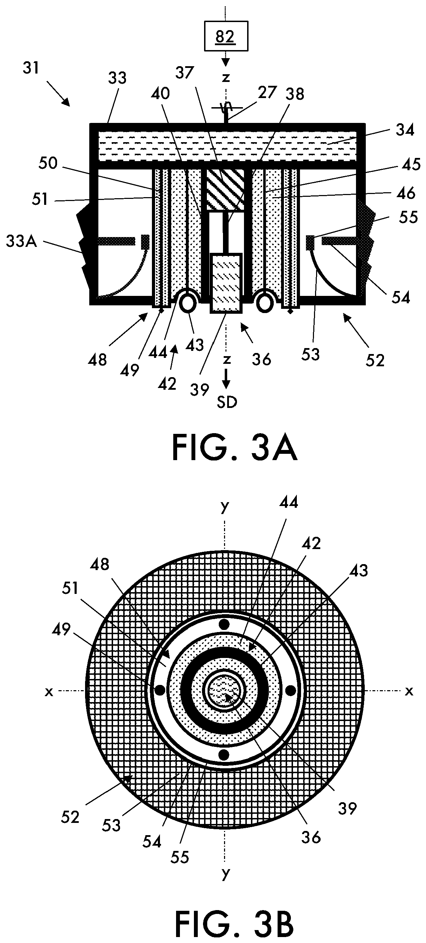

A cross-section of an exemplary energy generator 31 is depicted in FIG. 3A. As shown, each generator 31 may include: a housing 33; a controller 34; and a plurality of generator elements, such as: an impact or vibratory stimulus generator element 36; a thermal generator element 42; an electrical stimulus generator element 48; and a pressure generator element 52. Examples of each generator element are now described.

Similar to body 20, housing 33 may include an insulating material that surrounds portions of each generator 31 and/or defines mounting surfaces for generator elements 36, 42, 48, and/or 52. For example, housing 33 may be made of the same base material as body 20 or a compatible material; and/or formed together with body 20 by a molding, printing, or like process. As described below, portions of each generator element 36, 42, 48, and/or 52 may extend distally from housing 33 to contact the physiologic tissue (e.g., skin 2). Housing 33 of FIG. 3A includes an attachment feature 32 configured to secure each generator 31 in one of the communication bays 25. For example, attachment feature 32 may include a set of threads on housing 33 that are engageable with an interior surface of bays 25. Other types of chemical or mechanical attachment may be used, including biocompatible adhesives, snap-fit connections, and the like.

Exemplary generator elements 36, 42, 48, and 52 may be arranged to output their respective different energies 32 in approximately the same direction. As shown in FIGs. 3A and 3B, each generator element 36, 42, 48, and 52 may be arranged coaxially with communication axis z-z so that each energy 32 may be output toward the physiologic tissue (e.g., skin 2) in signal direction SD. Because of this coaxial configuration, each energy 32 may be output toward approximately the same point or area on a physiologic tissue (e.g., skin 2), making the energies 32 interchangeably communicable to the physiologic tissue. For example, any of the dots included in energy signal 90 of FIG. 1A may be interchangeably communicated to approximately the same point or area on skin 2 with any of the different energies 32.

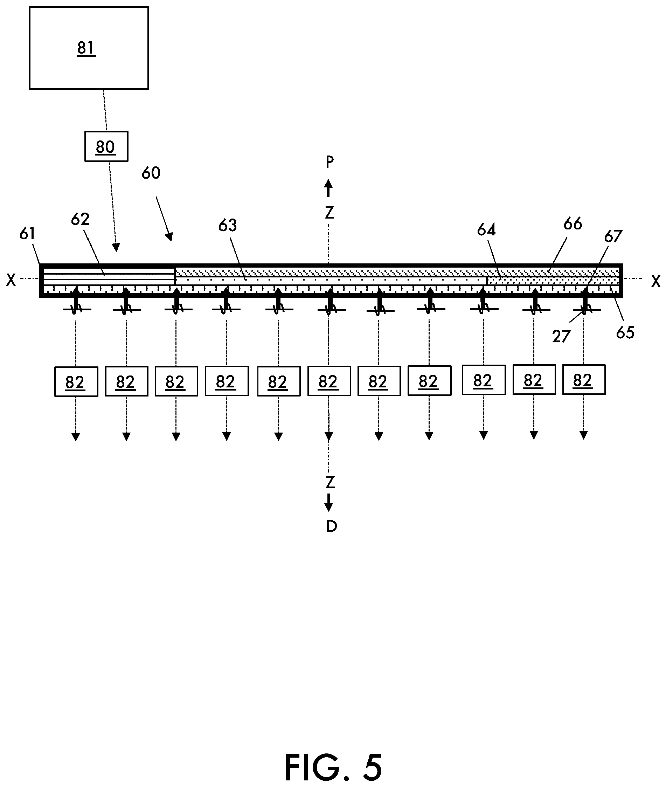

As shown in FIG. 3A, controller 34 may be configured receive a control signal 82 from processing unit 60, and activate generator elements 36, 42, 48, and 52 according to signal 82. The one or more conductors 27 may transmit the control signal 82 to generator elements 36, 42, 48, and 52 from processing unit 60 and/or direct electricity to generator elements 36, 42, 48, and 52 from a power source 66 of processing unit 60 (e.g., FIG. 5). Energy transceiver 10 may be an all-electrical device, wherein control signal 82 is an electrical signal and first and the conductors 27 are electrical wires. For varied response times, and energy requirements, transceiver 10 also may be an electro-optical device, wherein control signal 82 includes an optical signal, and at least one of the conductors 27 includes an optical fiber. For example, controller 34 may receive control signal 82 from processing unit 60 with a first one of conductors 27 (e.g., a first electrical and/or optical conductor), and direct electricity to one or more of the generator elements 36, 42, 48, and 52 with a second one of conductors 27 (e.g., a second electrical conductor) according to signal 82.

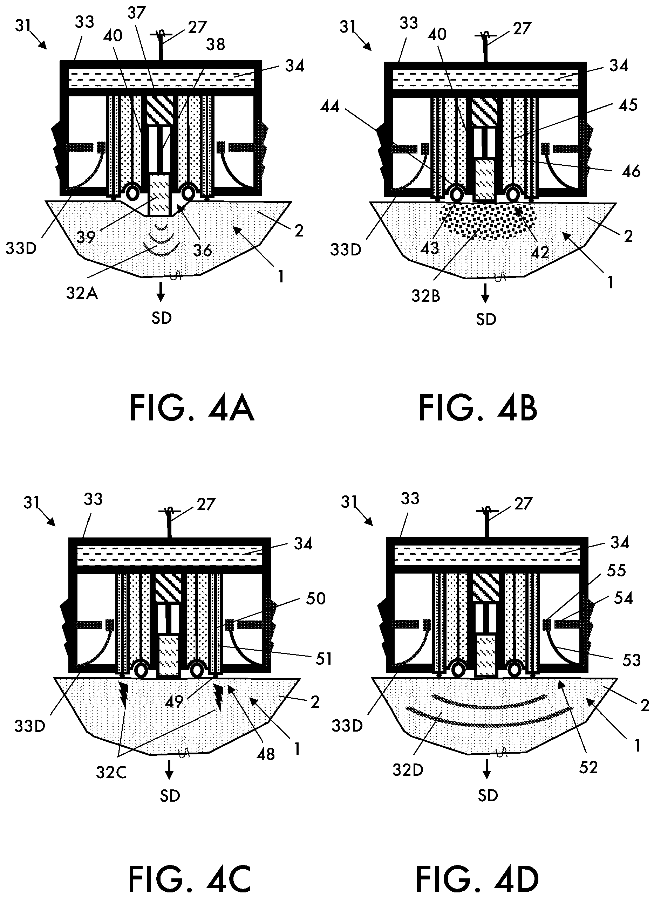

Additional aspects of generator elements 36, 42, 48, and 52 are now described with reference to FIGs. 4A-D. As shown in FIG. 4A, for example, impact or vibratory stimulus generator element 36 may communicate an impact or vibratory energy 32A to the brain through nerves associated with the skin 2. For example, impact or vibratory stimulus generator element 36 may comprise a mechanical actuator that converts electricity from power source 66 into a mechanical movement recognizable by touch receptors of skin 2, such as Meissner's corpuscle. As shown, generator element 36 may include a drive mechanism 37, a piston 38, a tissue contact 39, and a guide tube 40. Drive mechanism 37 may include a motor assembly that is attached to controller 34 and conductively engaged therewith. In this configuration, controller 34 may direct electricity to drive mechanism 37, causing the motor assembly to move piston 38 distally along communication axis z-z, outputting impact or vibratory energy 32A in signal direction SD. Different force transfer components also may be used to apply energy 32A, including levers and like actuators.

As shown, drive mechanism 37 may move piston 38 between a retracted position, wherein tissue contact 39 is contained housing 33 (e.g., FIG. 3A); and an extended position, wherein at least a portion of contact 39 is distal of housing 33 (e.g., FIG. 4A). Accordingly, impact or vibratory energy 32A may be output in signal direction SD as a physical movement of skin 2 caused by moving tissue contact 39 distally. Aspects of impact or vibratory energy 32A may be modified. For example, outer tube 40 may be attached to housing 33 and include interior surfaces configured to modify the timing of energy 32A by guiding the proximal-distal movements of tissue contact 39 (e.g., by rotating or stabilizing contact 39). A resilient element may be added between drive mechanism 37 and contact 39 to dampen such movements.

By way of example, impact or vibratory stimulus generator element 36 also may comprise a linear resonant actuator like those sold by Precision Microdrives Limited, such as their 6mm Linear Resonant Actuator having Model No. C12-003.001 and being available for sale at www.precisionmicrodrives.com.

Thermal generator element 42 may communicate a thermal energy 32B to the brain through nerves associated with skin 2. As shown in FIG. 4B, generator element 42 may include an electrical resistor that converts electricity from power source 66 into an amount of thermal energy recognizable by temperature receptors of skin 2 as being hot or cold, such the Ruffini corpuscle. For example, thermal generator element 42 may include an electrical resistor 43, a heat reflecting groove 44, a conductor 45, and an insulating material 46. Groove 44 may include a metal plate attached to an exterior surface of outer tube 40 of generator element 36. Resistor 33 may include an electrical wire or coil attached to groove 44. Conductor 45 may include an electrical wire extend between controller 34 and resistor 43, and material 46 may including an epoxy surrounding conductor 45.

As shown in FIG. 3B, electrical resistor 43 and heat reflecting groove 44 may be circular elements arranged coaxially with communication axis z-z. Conductor 45 may transmit electricity to electric resistor 43 for conversion into thermal energy 32B. Groove 44 may include a concave shape extending proximally into housing 33 to contain resistor 43, and the shape may include a distal surface configured to reflect heat energy 32B toward skin 2. In this configuration, thermal signal 32B may be output in signal direction SD as an amount of heat transferred to skin 2 by resistor 43. Aspects of thermal signal 32B may be modified. For example, the size, shape, and/or exterior coating of resistor 43 or groove 44 may modify the intensity of thermal energy 32B.

Electrical stimulus generator element 48 may communicate an electrical stimulus 32C to the brain through nerves associated with skin 2. As shown in FIG. 4C, electrical stimulus generator element 48 may comprise electrodes that convert electricity from power source 66 into an electrical stimulation recognizable by electricity-sensitive receptors of the physiologic tissue, such as the muscles and pain receptors located in the dermis layer of skin 2. For example, energy generator element 48 may include at least two electric contacts 49, conductors 50, and an insulating material 51. The conductors 50 may be metallic rods or wires extending distally from controller 34. Insulating material 51 may be an epoxy surrounding each conductor 50. Each contact 49 may include a discharge shape located on the distal-most end of one of conductors 50. In this configuration, controller 34 may direct electricity through conductors 50, and into the discharge shape of contact 49, allowing electricity to flow through skin 2 between the contacts 49 to output electrical stimulus 32C.

As shown in FIG. 3B, electrical contacts 49 may be spaced apart in a radial pattern coaxial with communication axis z-z. Any number of contacts 49 may be used, in any geometrical and/or spatial configuration. Insulating material 51 may be used to define and maintain the spacing. As shown, insulating materials 51 and 46 may be the same material, such as an epoxy. Four contacts 49 are shown in FIG. 3B, for example, as being arranged in two pairs. Aspects of electrical stimulus 32C may be modified. For example, the arrangement of contacts 49 may be changed; and/or the size of or spacing between each contact 49 changed to modify the intensity of electrical stimulus 32C.

Pressure generator element 52 may communicate a pressure energy 32D to the brain through nerves associated with skin 2. As shown in FIG. 4D, pressure generator element 52 may be an electroacoustic transducer that converts electricity from power source 66 into a sound wave recognizable by pressure receptors of skin 2, such as the Pacinian corpuscle. For example, pressure generator element 52 may include a cone 53, a voice coil 54, and a magnet 55. In this configuration, controller 34 may direct electricity into voice coil 54 for interaction with magnet 55, causing movements of cone 53 that generate the pressure energy 32D in signal direction SD.

As shown in FIGs. 3B and 4D, cone 53 may have a frustoconical shape that is coaxial with communication axis z-z. An outer edge of cone 53 may be attached an interior surface of housing 33, and an inner edge of cone 53 may be attached to voice coil 54, which may be coupled to controller 34 and power source 66 by one or more conductors. As shown, coil 54 may have a circular shape, and generator elements 36, 42, and 48 may be located in the interior of said shape. Aspects of pressure energy 32D may be modified. For example, cone 53 and/or voice coil 54 may include a surround, a spider, a secondary frame, or any other structures configured to modify signal responsiveness; the strength of magnet 55 may be varied; and/or controller 34 may include an amplifier configured to modify an intensity of pressure energy 32D.

Different generator element types also may be used to communicate signals to the skin with different energies 32, and/or different combinations of energies 32. For example, the plurality of generators 31 may be modified to vary individual or combined outputs of energies 32A, 32B, 32C, and 32D; and/or include additional generator elements configured to output additional signals to skin 2, including optical signals, magnetic signals, and/or any physically recognizable signals. Any type of generator element may be used and likewise coaxially arranged according to FIGs. 3A through 4D.

Additional aspects of an exemplary processing unit 60 are now described conceptually with reference to FIG 5. Any computing technologies may be utilized. As shown in FIG. 5, processing unit 60 may receive input data 80 from a data source 81 and output control signal 82 and/or electricity to each controller 34 via conductors 27, causing activation of one or more energy generators 31. For example, processing unit 60 of FIG. 5 includes a housing 61, a data transceiver 62, one or more processors 63, a memory 64, a communication bus 65, and a power source 66.

Data source 81 may include any combination of local and/or remote data sources that are in data communication with processing unit 60. For example, source 81 may include a local sensor that is located in one of communication bays 25 and configured to send input data 80 to unit 60 using conductors 27 and/or bus 65, allowing for closed loop communications in which energy signal 90 is based on data from the local sensors. Any sensing technologies may be used. For example, the local sensor may generate the input data 80 based on chemical and/or physical outputs related to skin 2.

Data source 81 also may include a remote data source in data communication with processing unit 60 via data transceiver 62, such as a remote sensor configured to send input data 80 to processing unit 60 with data transceiver 62 over a wired or wireless connection, allowing for open loop communications in which energy signal 90 is based on data from the local sensor and/or the remote sensor.

Any number and type of local sensors may be utilized to generate input data 80. The sensor(s) may be located at any position on or relative to energy transceiver 10 where they can be in data communication with processing unit 60. In the healthcare setting, for example, one local sensor may include a personal health tracker (e.g., a Fitbit^{®} or an iWatch^{®}) configured to generate input data 80 based on chemical and/or physical outputs of the wearer (e.g., heart rate, temperature), and communicate input data 80 to data transceiver 62 at regular intervals (e.g., once per second or once per minute).

Housing 61 may contain the elements of processing unit 60, and/or provide a means for removing processing unit 60 from body 2, allowing for easy repairs and upgrades. As shown in FIGs. 1B and 5, for example, exterior surfaces of housing 61 may be snap-fit with interior surfaces of compartment 23 so that the distal surface of processing unit 60 is maintained against the proximal surface of compartment 23. For example, the exterior surfaces of housing 61 of may include protrusions biased outwardly along the X-X and Y-Y axes, and the interior surfaces of compartment 23 may include grooves configured to receive said protrusions.

Transceiver 62 may include any wired or wireless communication technology configured to receive input data 80 form any data source(s) 81, such as Bluetooth, Wi-Fi, and the like. As shown in FIG. 5, input data 80 may be generated with or stored on data source 81 and received with transceiver 62. In a healthcare setting, for example, data source 81 may include at least one patient monitoring device configured to send input data 80 to a remote server at regular intervals (e.g., once per minute). Data 80 may include various measures regarding the patient, such as body temperature, pulse rate, respiration rate, and/or blood pressure. For example, transceiver 62 may retrieve and/or receive data 80 from the remote server at regular intervals (e.g., once per second or once per minute).

Each control signal 82 may be received with input data 80. Data transceiver 62 may relay the signals 82 to the one or more processors 63 and/or memory 64. Alternatively, processing unit 60 may generate each control signal 82 based on input data 80. For example, memory 64 may include a signal generating program, and one more processor 63 may generate each control signal 82 with the program. In keeping with previous examples, the signal generating program may be configured to: analyze the input data 80 sent from data sources 81 including a patient monitoring device during an interval; generate symbol 92A from the temperature and pulse rate, symbol 92B from the respiration rate, and symbol 92C from the blood pressure; and output a control signal 82 for communicating the symbols 92A, 92B, and 92C to skin 2.

As shown in FIG. 5, communication bus 65 may connect the one or more processors 63 and memory 64 to each generator 31, such as to each controller 34. Bus 65 may include electrical and/or optical connectors 67 located on and/or extending distally through housing 61. For example, communication bus 65 may comprise a flexible circuit board including a proximal surface supporting elements of processing unit 60, and a distal surface including an electrical and/or optical network extending from power source 66 to the connectors 67. Any type of network may be used, such as a mesh network. Connectors 67 may be engageable with corresponding connectors of conductors 27 to provide at least one pathway for outputting control signal 82 from processing unit 60 to one or more generators 31, and/or electricity from power source 66 to one or more generators 31. Control signal 82 may include electrical and/or optical signals. For example, control signal 82 may be include a string of output commands for each generator 31, and the entire string may be output to each generator 31 utilizing the electrical and/or optical signals, adding resiliency, in which the optical signals may be utilized for faster transmission.

As described above, the snap-fit connection between housing 61 and compartment 23 may place connectors 67 in communication with conductors 27, and maintain that communication over time, allowing for continuous output of control signals 82 from processing unit 60 and/or electricity from power source 66. A cover element may be attached to the proximal surface 24 of body 20 to seal processing unit 60 within compartment 23, and/or reinforce or supplant the snap-fit connection between housing 61 and compartment 23. For example, the cover may include a graphic design, a textual element, a writing surface, and/or like decorative feature. As a further example, the cover may provide a mounting surface for other technologies, such as an antenna, signal amplifier, and/or supplemental data transceiver.

Power source 66 may include any means for supplying electricity to processing unit 60 and/or the plurality of generators 31 (e.g., to each controller 34). As shown in FIG. 5, power source 66 may include a rechargeable battery, such as a lithium-ion battery, chargeable by connection to an external power source, such as a wall outlet. Power source 66 may include power generation technologies. For example, a proximal surface of power source 66 may include a power generator, such as photovoltaic cells configured to charge the battery. As shown in FIG. 5, power source 66 also may include an optical energy source, such as a laser generator that is powered by power source 66 and configured to output optical energy to one or more generators 31 via optical pathways defined by communication bus 65 and conductors 27.

Aspects of attachment element 70 are now described with reference to FIG. 2C. As shown, attachment element 70 may maintain a position of tissue interface 30 against or adjacent skin 2. For example, element 70 may include an adhesive, elastic, and/or fastening element configured to apply a maintaining force in signal direction SD. In FIG. 2C, element 70 includes a proximal surface 72 adhered to the distal surface 24 of body 20, and a distal surface 74 adherable with skin 2. Distal surface 74 of element 70 may include a biocompatible adhesive configured to apply the maintaining force.

Attachment element 70 may be removably and/or semi-permanently attached to skin 2 by the biocompatible adhesive. For example, a first adhesive material may be used to attach the proximal surface 72 to distal surface 24, and a second adhesive material may be used to attach distal surface 74 to skin 2. As a further example, the first adhesive may be stronger so that energy transceiver 10 may be removed from skin 2 without separating surfaces 72 and 24. Either the first or second adhesive material may be biocompatible and/or may include anti-bacterial and/or moisture resistant coatings and/or compositions configured for prolonged contact with skin 2. For example, at least the second adhesive material may be configured for contact with skin 2 during the entirety of a 4-hour, 8-hour, 12-hour, 24-hour shift, or longer shift. One or both adhesives also may be configured for semi-permanent contact with skin 2, such as during the entirety of a multi-month or multi-year treatment period. For example, at least the second adhesive material may include medicinal coatings and/or compositions that promote prolonged or semi-permanent contact with skin 2 by time-releasing treatments configured to prevent or minimize contact-based injuries.

Body 20 and/or attachment element 70 may boost the efficacy of energy signal 90 by minimizing and/or maintaining the distance between tissue interface 30 and skin 2, allowing signal 90 to be communicated with less energy. For example, any of the one or more different energies 32 may be output through body 20 and/or attachment element 70. As shown in FIGs. 2B and 2C, attachment element 70 may include a plurality of openings 76. Each opening 76 may be sized approximate to one of communication bays 25, allowing the energies 32 to be output towards skin 2 in signal direction SD through openings 76. For example, each opening 76 may have an inner diameter approximate to an outer diameter of the communications bay 25 or housing 33 for each generator 31. As shown in FIG. 2C, attachment element 70 may have a thickness that allows tissue contact 39, electrical resistor 43, and/or electrical contacts 49 to contact skin 2 through opening 76 or be adjacent to skin 2 within opening 76.

Aspects of body 20, housing 33, and/or attachment element 70 may direct and focus the energies 32, making it easier for the brain to distinguish one output of energies 32 from another. In keeping with previous examples, body 20, housing 33, and/or attachment element 70 of FIGs. 2B and 2C may be composed of base materials including an impact absorbing material configured to absorb any excessive vibrations of skin 2 caused by impact or vibratory energy 32A. One or both base materials may include an insulating material configured to direct thermal energy 32B, electrical stimulus 32C, and pressure energy 32D through openings 76 along axis Z-Z; and prevent transmission of stimulus 32B, 32C, and 32D along axis X-X and Y-Y. For example, body 20, housing 33, and/or element 70 of FIG. 2C may absorb any portion of energies 32 output incidentally in directions transverse to signal direction SD to promote signal distinction by limiting unwanted communications. As a further example, each opening 76 of attachment element 70 in FIG. 2C may have a reflective coating and/or a frustoconical interior shape centered about axis z-z to further focus the energies 32 towards skin 2.

As described herein, energy transceiver 10 may be operable to communicate energy signal 90 to skin 2 by outputting any energy 32, such as impact or vibratory energy 32A, thermal energy 32B, electrical stimulus 32C, and/or pressure energy 32D, individually or together. For example, any energies 32A-D may be used interchangeably or in combination to communicate any of the dots shown in FIG. 1A as symbols 92A, 92B, and 92C. As now described, aspects of each energy 32 may be modified to increase the complexity of signal 90, and thus the amount of data transmitted therewith. Modifiable aspects may include energy type, energy intensity, output duration, scroll rate, symbol shape, and the like.

Energy signal 90 may be communicated to skin 2 with energies 32, individually or together. In FIG. 1A, for example, each dot within first symbol 92A may be output with impact or vibratory energy 32A; each dot within second symbol 92B may be output with thermal energy 32B; and each dot within third symbol 92C may be output with electrical stimulus 32C. The energies 32 may be combined for additional emphasis. For example, the first symbol 92A may be output with impact or vibratory energy 32A in response to a baseline measure, and output with a combination of impact or vibratory energy 32A and thermal energy 32B if the measure changes. The energies 32 also may be combined to enhance the penetration depth of energy signal 90. For example, first symbol 92A may be formed by first outputting pressure energy 32D to activate a portion of the nerves associated with skin 2, and second outputting thermal energy 32B to the activated nerves. Any individual dot may be similarly modified relative to any other dot.

The intensity of energies 32 may be modified for emphasis. For example, processing unit 60 may output first symbol 92A with impact or vibratory energy 32A at a first intensity level in response to a baseline measure, and a second intensity level to highlight signal 92A if the measure changes. Output duration may be similarly modified. For example, the output duration of energies 32 may be instantaneous for normal measures, like a quick tap (e.g., about 100ms); extended for abnormal measures, like a short hold (e.g., 500ms to 1s); or a combination thereof, as with Morse code. Scroll rate may be similarly modified. For example, symbols 92 may not be scrolled at all (i.e., a scroll rate of zero), and output duration may be used to communicate change over time by flashing symbols 92 off and or in a fixed position. As a further example, in the healthcare setting, the scroll rate may be based on an update schedule (e.g., one revolution per minute), and/or the output duration may be based on patient status (e.g., faster for more critical patients).

Symbol shape also may be modified. The plurality of symbols 92 are shown as pip pattern shapes in FIG. 1A, but any symbol shape may be used, particularly those amenable to dot-matrix representation. For example, the plurality of symbols 92 may include known Morse code, binary symbols, lines, and/or directional arrows that are scrolled across communication area 4 in communication direction CD. Alphanumeric symbols also may be communicated. For example, input data 80 may include a control signal 82 generated from a Twitter^{®} feed, and the symbols 92 may include alphanumeric symbols for communicating the author, date, and content of each Tweet^{®} contained in the feed. As a further example, input data 80 may include the subject and sender of an email, and the signal generating program included in memory 64 may be configured to: prioritize the email based on the sender; and generate a control signal 82 for outputting a set symbols 92 based on the subject, sender, and priority of the email. For example, first symbols 92 may be output with impact or vibratory energy 32A to communicate the subject and/or sender of prioritized emails in a shorthand notation, and at least one of thermal energy 32B, electrical stimulus 32C, pressure energy 32D to communicate the priority level of the shorthand notation.

The resolution of tissue interface 30 may match or exceed the distinguishing capabilities of the nerves associated with skin 2. For example, in the grid formation shown in FIG. 2B, the resolution of tissue interface 30 may be measured as energy output per square inch (m²), which may exceed the natural energy receptivity limits of the nerves associated with skin 2. As shown, the resolution of interface 30 may be relative to the spacing between each bay 25, the configuration of body 20 and/or attachment element 70, and/or the intensity of energies 32. The energy receptivity limits of skin 2 may vary by location. For example, energy transceiver 10 may be attached to a portion of skin 2 located in a highly innervated or sensitive area, such as the face, allowing even more complex symbol shapes to be communicated.

With sufficient resolution, tissue interface 30 may output energy signal 90 to replicate image patterns and/or other sensory perceptions with energies 32, including any of the symbols described herein and even more complex interactions. As described herein, the multi-energy capabilities of energy transceiver 10 may be utilized to layer outputs of different energies 32 so as to communicate far more complex image patterns and/or sensory perceptions that would otherwise be possible by communicating with a single energy because of the natural receptivity limits of the nerves, and their tendency to become less receptive during prolonged exposures.

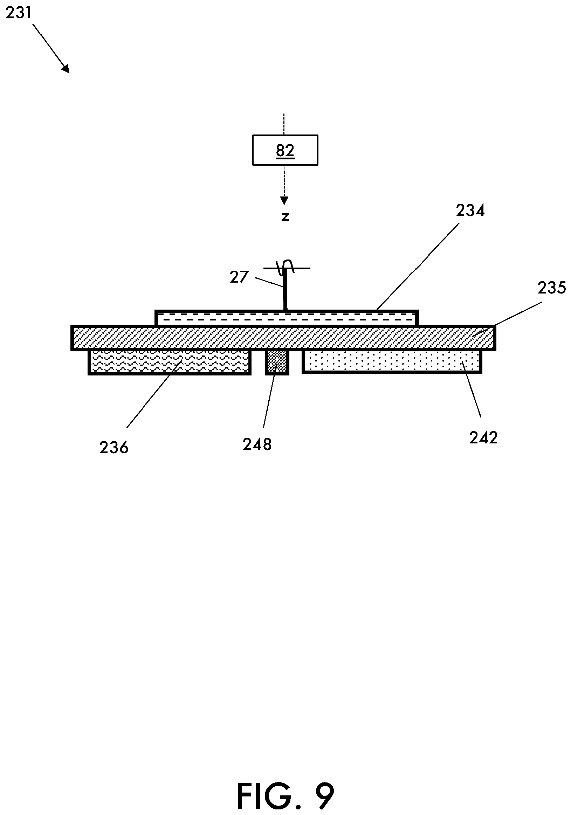

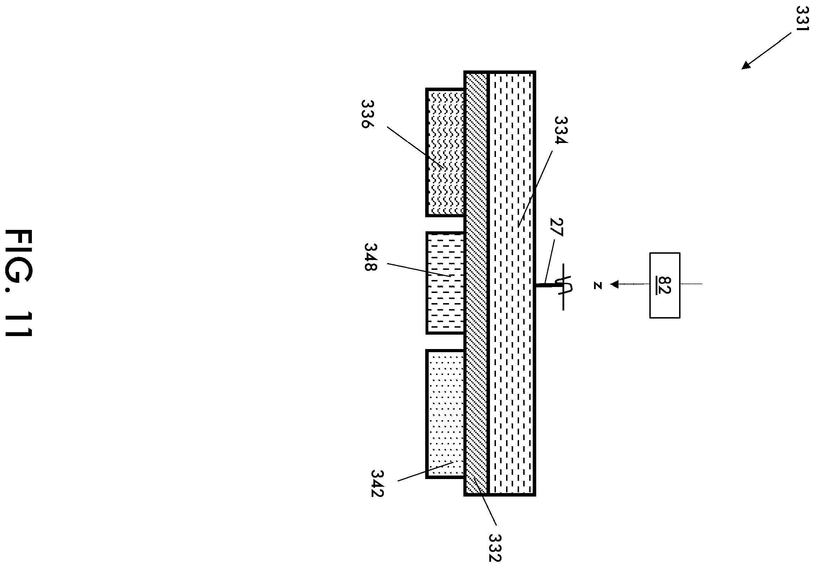

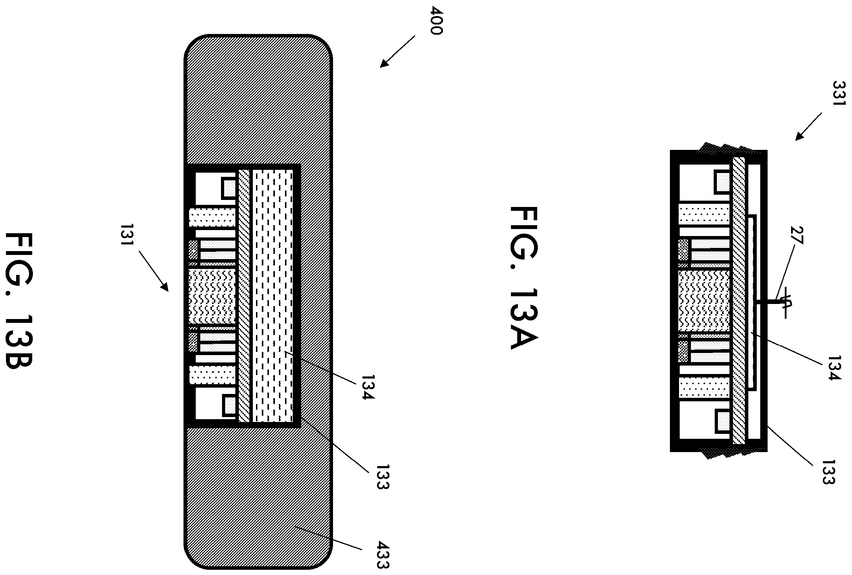

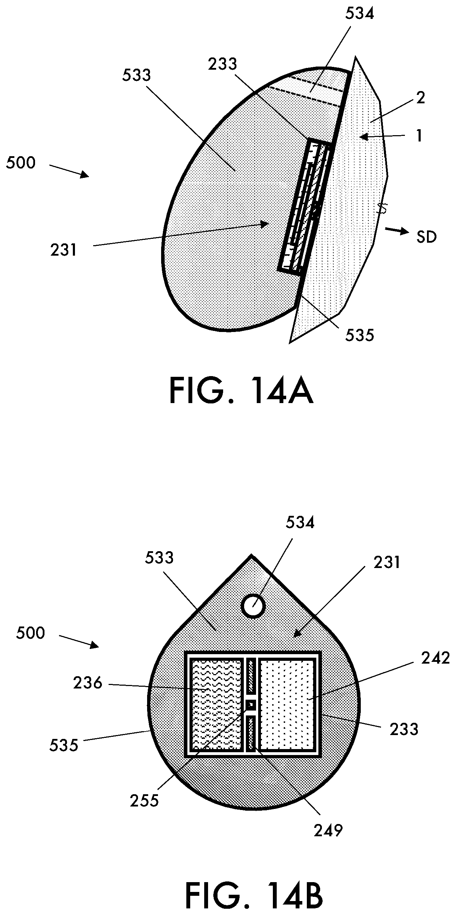

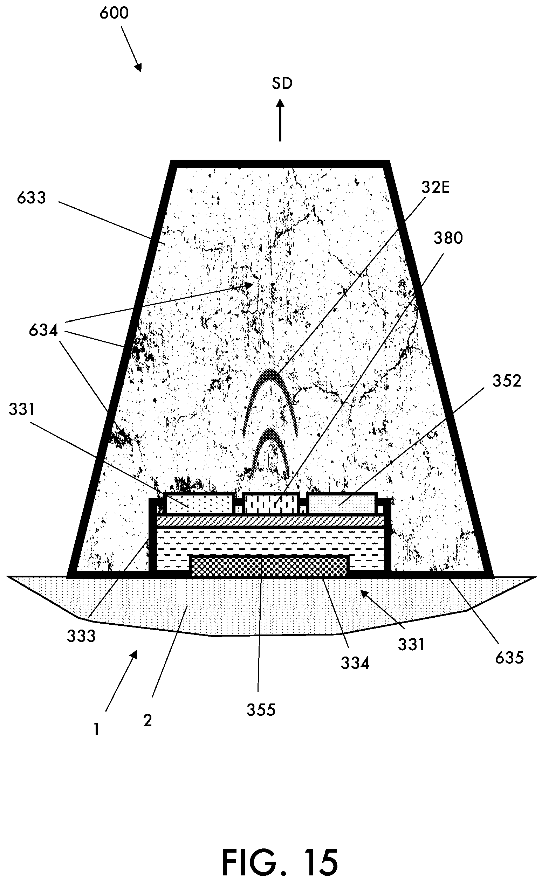

Additional aspects of this disclosure are now described with reference to additional examples of energy generator 31, including: an exemplary energy generator 131 shown conceptually in FIGs. 6, 7, 8A, 8B, 8C, 8D, 13A, and 13B; an exemplary energy generator 231 shown conceptually in FIGs. 9, 10, 14A, and 14B; and an exemplary energy generator 331 shown conceptually in FIGs. 11, 12, and 15. A number of related examples are described with reference to generators 131, 231, and 331, including: an exemplary data communication apparatus 400 shown conceptually in FIG. 13B; an exemplary data communication apparatus 500 shown conceptually in FIGs. 14A and 14B; and an exemplary data communication apparatus 600 shown conceptually in FIG. 15.

Each variation of energy generator 31, such as generators 131, 231, and 331, may include elements similar to those of generator 31, but within the respective 100, 200, or 300 series of numbers, whether or not those elements are depicted in FIGs. 6 through 15. Any aspects described with references to generators 131, 231, and 331 may be included within any variation of generator 31 described herein, each possible combination or iteration being part of this disclosure. For example, any examples of energy generator 31, 131, 231, 331 described herein may comprise any energy generating elements described with reference any such examples.

In contrast to energy generator 31 shown in FIGs. 3A and 3B, energy generator 131 of FIGs. 6 and 7 may comprise a thinner construction and additional capabilities. As shown in FIG. 6, energy generator 131 may comprise: a frame 132; a housing 133; a controller 134; and a plurality of generator elements, such as: an impact or vibratory stimulus generator element 136; a thermal generator element 142; a shock or electrical stimulus generator element 148; and a pressure generator element 152. Examples of each are now described.

Energy generator 131 may be sold with or without housing 133, making it an optional element. Energy generator 131 is shown without housing 133 in FIGs. 6 and 7 and with housing 133 in FIGs. 13A and 13B. Where shown, housing 133 of energy generator 131 may be a counterpart to housing 33 of energy generator 31 described above. For example, housing 133 may be made of the same base material and similarly formed to absorb or deflect portions of energies 32 output incidentally in directions transverse to signal direction SD.

Energy generator 131 may be incorporated into another electronic device such as data communication device 400. As shown in FIG. 13B, exterior surfaces of housing 333 may optionally be embeddable in a graspable body 433 of data communication device 400 that is operable to generally maintain a position of energy generator 131 in a hand of user 1 when grasping body 433. As shown in FIG. 13B, graspable body 433 may comprise cylindrical, coin-shaped structure made from a polymeric material that is radio translucent with respect to Wi-Fi. For example, body 433 may comprise a rubberized polymer designed to provide a reliable grip surface for data communication device 400, provide fall protection for energy generator 131, and limit energies 32 in directions transverse to signal direction SD.

As shown in FIG. 6, frame 132 comprises a printed circuit board (or "PCB") operable to mechanically support and electrically connect controller 134 and the plurality of generator elements, including impact or vibratory stimulus generator element 136, thermal generator element 142, shock or electrical stimulus generator element 148, and pressure generator element 152. For example, frame 132 may comprise conductive tracks, pads and other features etched from one or more sheet layers of copper laminated onto and/or between sheet layers of a non-conductive substrate. As shown in FIG. 6, controller 134 and elements 136, 142, 148, and 152 may be soldered onto frame 132 to electrically connect and mechanically fasten them to a common platform.

Frame 132 may have perimeter edges engageable with interior surfaces of housing 133 to position controller 134 and elements 136, 142, 148, and 152 in housing 133 or another structure. As shown in FIG. 6, the perimeter edges of frame 132 may be located in an annular recess formed into housing 133 to define a proximal cavity of housing 133 positioned below frame 132 and a distal cavity of housing 133 positioned above frame 132. The plurality of generator elements and edges of frame 132 may comprise seals and/or sealing elements that prevent moisture from entering the proximal and distal cavities of housing 133 and/or otherwise interfering with controller 134, helping to seal it off in the distal cavity.

Controller 134 may be a counterpart of controller 34 of energy generator 31. For example, similar to as shown in FIGs. 3A and 5, controller 134 may be configured receive or generate control signal 82 and activate any one or more of generator elements 136, 142, 148, and 152 according to control signal 82. Conductors 27 may transmit the control signal 82 and electricity to controller 134 for distribution to generator elements 136, 142, 148, and/or 152 when activated. Controller 134 may be modified to include any generator elements, such as by adding additional elements for computing, thermal management, and/or power management.

As above, generator elements 136, 142, 148, and 152 may be arranged to output their respective different energies 32 in approximately the same direction relative to signal direction SD. As shown in FIGs. 6 and 7, each generator element 136, 142, 148, and 152 may be arranged coaxially so that each different energy 32 may be output toward the nerves associated with the physiologic tissue (e.g., skin 2) in a common signal direction SD. Because of this coaxial configuration, each different energy 32 may be output toward approximately the same point or area on skin 2.

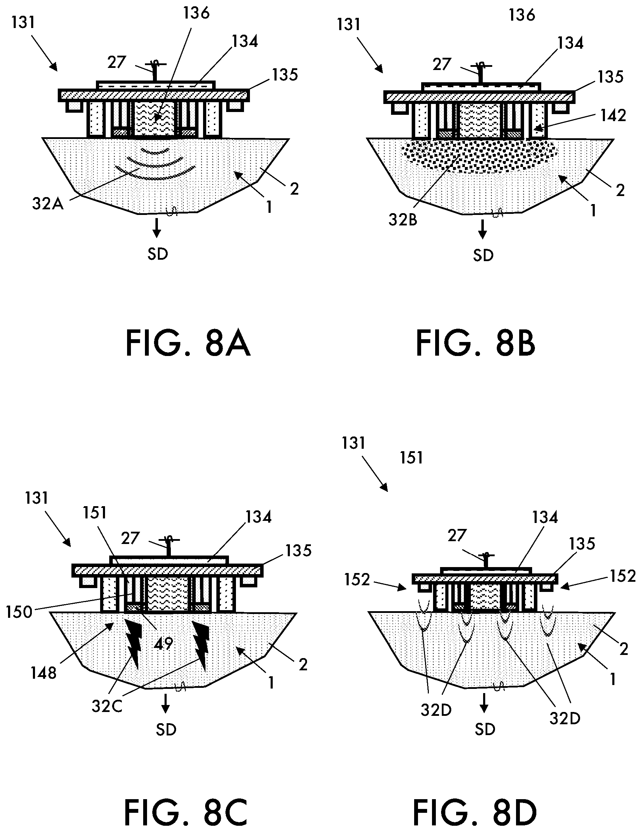

Additional aspects of generator elements 136, 142, 148, and 152 are now described with reference to FIGs. 6, 7, 8A, 8B, 8C, and 8D. As shown in FIGs. 6 and 8A, impact or vibratory stimulus generator element 136 may communicate an impact or vibratory energy 32A to the brain through nerves associated with the physiologic tissue (e.g., skin 2). As shown in FIG. 8A, impact or vibratory energy 32A may be output to the physiologic tissue (e.g., skin 2) in signal direction SD when a contact surface of impact generator element 136 is maintained against the tissue. For example, impact or vibratory stimulus generator element 136 (like element 36) may comprise a mechanical actuator that converts electricity into a mechanical movement recognizable by touch receptors of skin 2, such as Meissner's corpuscle.

As shown in FIGs. 6 and 7, a circular housing of impact or vibratory stimulus generator element 136 may define a connection interface that is structurally and electrically mountable on a pad of frame 132 to place generator element 136 in electrical communication with controller 134, allowing for transmission of data and/or power. generator element 136 may comprise a coin vibration motor, shaftless motor, pancake vibrator motors, or any other motor with an enclosed vibration mechanism and circular shape. By way of example, impact or vibratory stimulus generator element 136 may comprise a coin vibration motor like those sold by Precision Microdrives Limited, such as their Pico Haptic Shaftless Vibration Motor available for sale at www.precisionmicrodrives.com.

As shown in FIGs. 6, 7, and 8B, thermal generator element 142 may communicate a thermal energy 32B to the brain through nerves associated with the physiologic tissue (e.g., skin 2). As shown in FIGs. 6 and 7, thermal generator element 142 may convert electricity into an amount of thermal energy recognizable as hot or cold by temperature receptors of skin 2, such the Ruffini corpuscle. As shown in FIG. 7, thermal generator element 142 may comprise a flexible thermoelectric generator that utilizes the Seebeck effect to create a temperature differential responsive to an electric current supplied to thermal generator element 142 by controller 134. The temperature differential may be perceivable by the nerves associated with the physiologic tissue (e.g., by the Ruffini corpuscle) as different degrees and variable experiences of hot and cold. Rapid oscillations of thermal energy in the form of hot and cold cycles also may be realized, creating additional thermal effects.

As shown in FIG. 7, thermal generator element 142 may comprise an annular shape that surrounds impact or vibratory stimulus generator 136 and shock or electrical stimulation generator 142. As shown in FIGs. 6 and 8B, the annular shape of element 142 may define a continuous body with a wide tissue contact surface operable to evenly output thermal energy 32B in signal direction SD toward the physiologic tissue (e.g., skin 2). In many areas of skin 2, for example, the density of Ruffini corpuscles may allow user 1 to experience thermal energy 32B as a continuous output despite the annular shape of thermal generator element 142. Thermal generator element 142 may comprise a flexible thermoelectric generator having an annular housing and a connection interface that is structurally and electrically mountable on a pad of frame 132 to place thermal generator element 142 in electrical communication with controller 134, allowing for transmission of data and/or power. By way of example, thermal generator element 42 may comprise a flexible thermoelectric generator such as those sold by TEGway at www.tegway.co.

As shown in FIGs. 7 and 8C, shock or electrical stimulus generator element 148 may communicate an electrical stimulus 32C to the brain through nerves associated with skin 2. As shown in FIG. 6, electrical stimulus generator element 148 may comprise electrode plates that convert electricity into an electrical stimulation recognizable by electricity-sensitive receptors of the physiologic tissue, such as the muscles and pain receptors located in the dermis layer of skin 2. As shown in FIGs. 6 and 8D, electrical stimulus generator element 148 may comprise a pair of contact plates 149, conductors 150, and an insulating material 151. The conductors 150 may comprise metallic rods or wires extending distally from controller 134. Insulating material 151 may comprise an epoxy that surrounds each conductor 150 and defines a structural support interface for pair of contact plates 149 that connects them to frame 132.

Similar to above, each contact plate 149 may include a discharge shape located on the distal-most end of one of conductors 150 and insulating material 151, allowing controller 134 to direct electricity through conductors 150, into the discharge shapes of contact plates 149, and through skin 2. In contrast to above, as shown in FIG. 7, each contact plate 149 may comprise a partial annular shape, like a half-moon shape, which may be located between impact or vibratory generator element 136 and thermal generator element 142. The increased contact area of plates 149 may allow for increased amounts of electrical stimulus 32C to be output with energy generator 131.

By way of example, electrical stimulus generator element 48 may comprise any type of electrode and/or contact plate operable to apply electrical stimulation to the physiologic tissue (e.g., skin 2), such as those sold under the name Relief Band at www.reliefband.com and described in U.S. Patent No. 7,893,761 .

Pressure generator element 152 may communicate a pressure energy 32D to the brain through nerves associated with skin 2. As shown in FIGs. 7 and 8D, pressure generator element 152 may convert electricity into a sound wave recognizable by pressure receptors of skin 2, such as the Pacinian corpuscle. Pressure generator element 152 may be optimized to reduce a thickness of energy generator 131 relative to that of energy generator 31 (e.g., FIG. 3A). For example, each piezoelectric speaker 153 may comprise a perimeter structure mounted to a pad of frame 132, a diaphragm moveable relative to the perimeter structure, and a piezoelectric ceramic sheet operable to covert the electricity into movements of the diaphragm that cause pressure energy 32D in the form of a sonic energy (e.g., sound waves and/or pressures) output toward the physiologic tissue (e.g., skin 2) through an air gap located between speaker 153 and the tissue.

As shown in FIG. 6, pressure generator element 152 may comprise a plurality of piezoelectric speakers 153 arranged in a radial array that is coaxially with communication axis z-z. In this arrangement, each pressure generator element 152 may independently output a different portion of pressure energy 32D in signal direction SD toward the physiologic tissue (e.g., skin 2). Each piezoelectric speaker 153 may comprise a connection interface that is structurally and electrically mountable on a pad of frame 132 to place generator element 152 in electrical communication with controller 134, allowing for transmission of data and/or power. In many areas of skin 2, for example, the density of Pacinian corpuscles may allow user 1 to experience the different portions of pressure energy 32D as a continuous effect despite the annular shape of element 142.

Piezoelectric speakers 153 may be operable to output any frequencies and sound pressures perceivable by the nerves associated with the physiologic tissue (e.g., skin 2). As shown in FIGs. 6 and 8D, one or more of piezoelectric speakers 153 may additionally or alternatively comprise an ultrasound transducer operable to output ultrasound waves for diagnostic and/or treatment purposes when energy generator 131 is maintained against the skin. For example, each piezoelectric speaker 153 may comprise a piezoelectric ceramic speaker, such as those sold by the TDK Corporation (www.tkd.com) under the name PiezoListen ^{Tll} and known to have an operating frequency range of 400 to 20,000 Hz and sound pressure of 80 dB. In this example, pressure generator element 152 may communicate waves to the physiologic tissue (e.g., skin 2) for purposes of communicating with user 1 and/or sensing physiological signals of user 1.

As above, different generator elements of energy generator 131 may be activated to by controller 134 to output one or more different energies 32, and/or different combinations of the different energies 32. For example, any of generator elements 136, 142, 148, and/or 152 may be modified according to any examples described herein to vary any individual or combined outputs of energies 32A, 32B, 32C, and 32D.

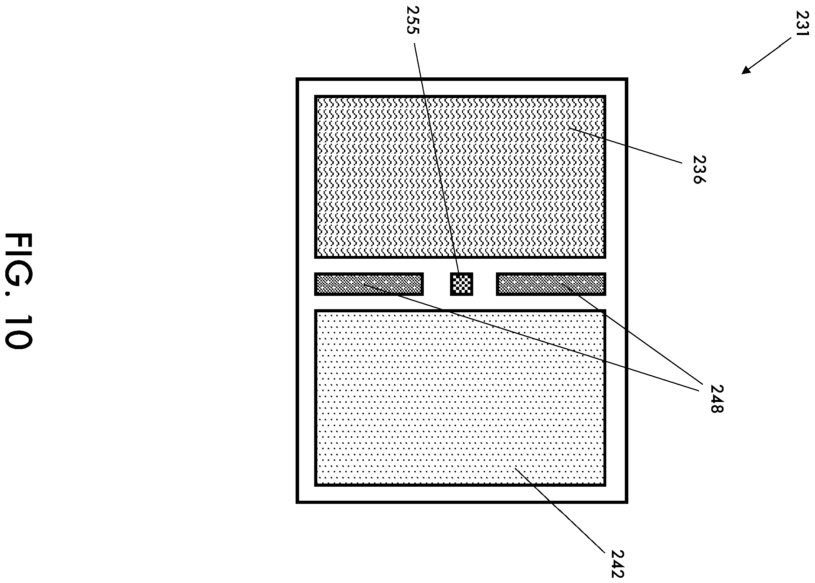

Energy generator 231 of FIGs. 9 and 10 may comprise an alternative construction that is comparatively thinner than the construction of energy generator 131 of FIGs. 6 and 7. As shown in FIGs. 9, 10, 14A, and/or 14B, energy generator 231 may comprise: a frame 232; a housing 233; a controller 234; a plurality of generator elements, such as an impact or vibratory stimulus generator element 236, a thermal generator element 242, and a shock or electrical stimulus generator element 248; and a sensor 255. Examples of each are now described.

Frame 232 and housing 233 of energy generator 231 may be similar to frame 132 and housing 133, except that edges of frame 232 may comprise a rectangular perimeter and interior surfaces of housing 233 may comprise a rectangular groove engageable with the edges of frame 232.

Energy generator 231 may be incorporated into another electronic device such as data communication device 500. As shown in FIGs. 14A and 14B, exterior surfaces of housing 233 may optionally be embeddable in a wearable body 533 of data communication device 500 that is operable to generally maintain a position of energy generator 431 when worn on user 1. Wearable body 533 may comprise a decorative material having established value and/or associated affective properties, such as any crystalline structure and/or a polymeric structure, including the exemplary rose quartz crystal structure shown conceptually in FIGs. 14A and 14B. For example, wearable body 533 may comprise an upper portion with an opening 534 sized to receive a cord or chain adapted to support wearable body 533 around a neck of user 1 so that a skin-facing surface 535 of wearable body 533 may be maintained against an area of skin 2 on the chest of user 1 by gravity forces applied by wearable body 533.

Portions of housing 233 may conduct and/or transfer portions of one or more different energies 32 to wearable body 533, allowing body 533 to absorb and store some of energies 32. As shown in FIG. 14A, housing 233 may comprise a metallic structure comprising exterior surfaces with attachment features (e.g., roughened areas and/or grooves or ridges) that are engageable with interior surfaces of an opening formed in wearable body 533. As shown in FIGs. 14A and 14B, the hole may be drilled and/or laser cut to receive housing 233 and a conductive epoxy may be utilized to engage the exterior surfaces of housing 233 with interior surfaces of the hole.

Controller 234 may be a counterpart to controllers 34 and 134 described above and thus similarly operable to receive or generate control signal 82 and activate any one or more of generator elements 236, 242, and 248 according to control signal 82. As shown in FIGs. 14A and 14B, controller 234 may comprise additional elements for configuring data communication apparatus 500 to operate independently as a standalone device, including those of processing unit 60.

In keeping with above, energy generator elements 236, 242, and 248 may be arranged to output their respective different energies 32 in approximately the same direction, such that all of the energies 32 may be output in signal direction SD albeit at different locations on frame 232. As shown in FIGs. 9 and 10, each generator element 236, 242, and 248 may be arranged adjacent to one another so that their respective communication directions are parallel with communication axis z-z so that each different energy 32 may be output toward the nerves associated with the physiologic tissue (e.g., skin 2) in signal direction SD. Because of this adjacency, different energies 32 may be output toward adjacent points or areas on skin 2.

Additional aspects of generator elements 236, 242, and 248 are now described with reference to FIGs. 9, 10, 14A, and 14B. As shown in FIG. 9 and 14A, impact or vibratory stimulus generator element 236 may communicate an impact or vibratory energy 32A to the brain through nerves associated with the physiologic tissue (e.g., skin 2). As shown in FIG. 14A, impact or vibratory energy 32A may be output to the physiologic tissue (e.g., skin 2) when a contact surface of impact or vibratory stimulus generator element 236 is maintained against the tissue, such as when contact surface 535 of weighted body is maintained against skin 2 by gravity forces applied by wearable body 533. Impact or vibratory stimulus generator element 236 may similarly comprise a coin vibration motor in a rectangular housing. As shown in in FIG. 6, the rectangular housing of generator element 236 may define a connection interface that is structurally and electrically mountable on a pad of frame 232 to place element 236 in electrical communication with controller 234, allowing for transmission of data and/or power.

Thermal generator element 242 may communicate a thermal energy 32B to the brain through nerves associated with the physiologic tissue (e.g., skin 2). As shown in FIGs. 9 and 10, thermal generator element 242 may be a counterpart to thermal generator elements 42 and/or 142 and thus similarly configured to convert electricity into an amount of thermal energy recognizable as hot or cold by temperature receptors of skin 2, such the Ruffini corpuscle. As shown in FIG. 14A, impact or vibratory energy 32A may be output to the physiologic tissue (e.g., skin 2) in signal direction SD when a contact surface of thermal generator element 242 is maintained against the tissue. As shown in FIGs. 9 and 14B, thermal generator element 242 may comprise a flexible thermoelectric generator having a rectangular housing and a connection interface that is structurally and electrically mountable on a pad of frame 232 to place thermal generator element 242 in electrical communication with controller 234, allowing for transmission of data and/or power.

As shown in FIGs. 9, 10, 14A, and 14B shock or electrical stimulus generator element 248 may communicate an electrical stimulus 32C to the brain through the nerves associated with the physiologic tissue (e.g., skin 2). As shown in FIG. 9, electrical stimulus generator element 248 may comprise contact a pair of plates 249 that are spaced apart from one another (e.g., between generator elements 236 and 242) and operable to convert electricity into an electrical stimulation recognizable by electricity-sensitive receptors of the physiologic tissue, such as the muscles and pain receptors located in the dermis layer of skin 2. As shown in FIGs. 9 and 14, each contact plate 249 may comprise a connection interface that is structurally and electrically mountable on a pad of frame 232 to place electrical stimulus generator element 248 in electrical communication with controller 234, allowing for transmission of data and/or power.

Similar to above, each contact plate 249 of electrical stimulus generator element 248 may comprise a discharge shape located on frame 232, allowing controller 34 to direct electricity into the discharge shapes of contact plates 249. In contrast to above, as shown in FIG. 9, each contact plate 249 may mounted on or adjacent frame 232 with little or no additional insulating material to further reduce a thickness of energy generator 231 relative to energy generators 31 and 131, which may utilize small air gaps between pressure generators 52, 152 and the physiologic tissue (e.g., skin 2) to affect communication of pressure energy 32D to the tissue.

As shown in FIGs. 10 and 14A, sensor 255 may measure and output physiological data about user 1, including any brain sensors and/or body sensors adapted to measure energies output directly to and/or indirectly from user 1. For example, sensor 255 may comprise any combination of known sensing technologies, such as: a heart sensor (e.g., PPG + Pulse Oximtery) adapted to output heart signals for user 1; a body sensor (e.g., IMU) adapted to output motion signals for user 1; and a breath sensor (e.g., PPG + Gyroscope) adapted to output breath signals for user 1. As shown in FIG. 14A, depending upon its position on the physiologic tissue (e.g., skin 2), sensor 255 may compromise any sensing technology capable of generating measurement data for user 1 based on their body's electrical activity, such as that measurable in relation to their heart (e.g., ECG), muscle (e.g., EMG). Different combinations of measures such as PPG and ECG may be used to offer greater accuracy and decrease noise.

As shown in FIGs. 10 and 14B, sensor 255 may serve as a data source for data communication apparatus 500. For example, sensor 255 may be operable with controller 234 to output sensory data to processing unit 60, which may be operable with lines of code to responsively generate and/ or output a control signal 82 causing one or more of generator elements 236, 242, and/or 248, when positioned relative to the physiologic tissue (e.g., skin 2), to communicate with different nerves associated with the tissue by outputting a different portion energy signal 90 in signal direction SD toward the tissue with one energy type of the plurality of different energies 32.

Because of the energy communication between housing 233 and wearable body 533, and further because of the data and power communication between sensor 255 and processing unit 60, data communication apparatus 500 may thus be operable as a standalone data reactive device providing user 1 with a different way to consume data. As described herein, energy signal 90 may be utilized to both communicate energies 32 to the physiologic tissue (e.g., skin 2) and apply portions of energies 32 to wearable body 533 for the purpose of charging it with thermal energy and/or causing it to vibrate with element 236, making wearable body 533 an amplifying element for energy generator 531 that helps user 1 to further experience and engage the data.

Energy generator 331 may comprise another alternative construction with additional output capabilities. As shown in FIGs. 11, 12, and/or 15, energy generator 331 may comprise: a frame 332; a housing 333; a controller 334; a plurality of generator elements, such as: an impact or vibratory stimulus generator element 336; a thermal generator element 342; an optical energy generator 348; and a sensor 355. Examples of each are now described.

Frame 332 (e.g., FIG. 11) and housing 333 (e.g., FIG. 15) of energy generator 331 may be counterparts to frame 232 (e.g., FIG. 9) and housing 233 (e.g., FIGs. 14A and 14B) of energy generator 213.

Energy generator 331 may be incorporated into another electronic device such as data communication device 600. As shown in FIG. 15, exterior surfaces of housing 333 may optionally be embeddable in a self-supporting body 633 of data communication device 600 that is operable to generally maintain a position of energy generator 331 when placed on a horizontal surface, such as a generally planar portion of the physiologic tissue (e.g., skin 2) of user 1. Much like wearable body 533, self-supporting body 633 may comprise a decorative material having established value and/or associated affective properties, such as any crystalline structure and/or a polymeric structure, including the composite polymeric material with additives 634 described further below. For example, self-supporting body 633 may comprise an elongated shape defining a grip surface extending upward from a support surface 635 operable to prevent data communication apparatus 600 from falling over when at rest.

Much like body 20 and housing 33, housing 333 may have insulating and/or energy-directing properties. For example, a base material of housing 333 may include compositions and/or coatings that promote energy flows in directions parallel to signal direction SD and limit energy flows in directions that are perpendicular to signal direction SD. Housing 333 may be similarly manufactured using any known process, such as molding or 3D printing with a base material that is biocompatible, dielectric, impact resistance, sound absorbing, and/or thermally resistant, to have exterior surfaces engageable with interior surfaces of supporting graspable body 633. As shown in FIG. 15, a hole may be drilled and/or laser cut into support surface 335 to receive housing 333 so that generator elements 336, 342, and 348 may be positioned toward an interior portion of self-supporting body 633 and thus operable to output their respective different energies 32 into and through self-supporting body 633.

Controller 334 may be a counterpart to controllers 34, 134, and 234 described above and thus similarly operable to receive or generate control signal 82 and activate any of generator elements 336, 342, and 348 according to signal 82. As shown in FIG. 15, controller 334 may comprise additional elements for configuring data communication apparatus 600 to operate independently as a standalone device, including those of processing element 60. Controller 334 also may comprise additional heat transfer elements operable with optical generating element 248 to prevent overheating by transfer any excess heat into interior portions of self-supporting body 633.

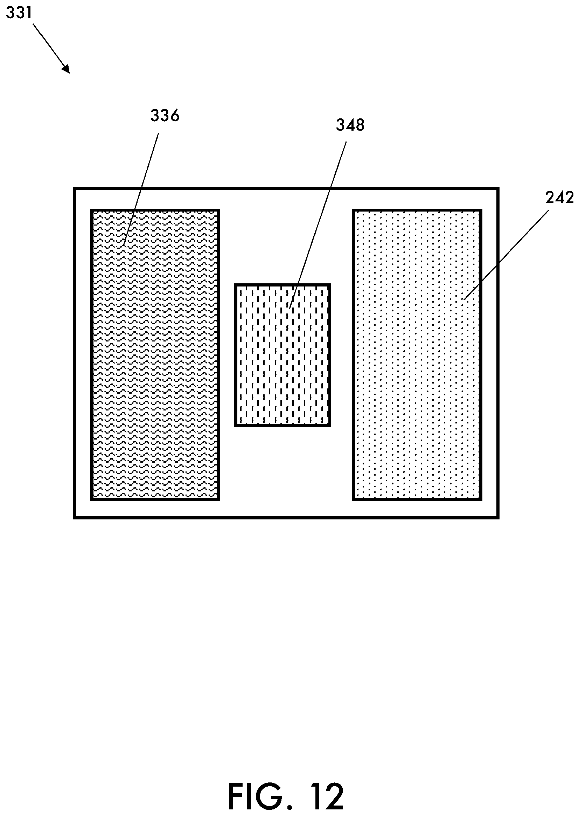

In keeping with above, energy generator elements 336, 342, and 348 may be arranged to output their respective different energies 32 in approximately the same direction, such that all of the energies 32 may be output in signal direction SD albeit at different locations on frame 332. As shown in FIGs. 11 and 12, each generator element 336, 342, and 348 may be arranged adjacent to one another so that their respective communication directions are parallel to one another so that each different energy 32 may be output toward the nerves associated with the physiologic tissue (e.g., skin 2) in a similar signal direction SD when energy generator 331 is incorporated into a wearable or implantable technology like energy transceiver 10. Alternatively, as shown in 15, each generator element 336, 342, and 348 also may be arranged adjacently so that different energies 32 are output toward the interior portion of self-supporting body 633 in a similar signal direction SD when energy generator 331 is incorporated into data communication apparatus 600.

Additional aspects of generator elements 336, 342, and 348, are now described with reference to FIGs. 11, 12, and 15. As shown in FIG. 11, impact or vibratory stimulus generator element 336 may communicate an impact or vibratory energy 32A to the brain through nerves associated with the physiologic tissue (e.g., skin 2). As shown in FIG. 15, impact or vibratory energy 32A may be output to the physiologic tissue (e.g., skin 2) when support surface 635 of self-supporting body 633 is maintained against the tissue by gravity forces. For example, impact or vibratory stimulus generator element 336 may output impact or vibratory energy 32A to skin 2 when self-supporting body 633 is grasped in a hand of user 1 and/or when support surface 635 is resting on a generally planar portion of user such as their forehead. In keeping with above, impact or vibratory stimulus generator element 336 may similarly comprise a coin vibration motor in a rectangular housing. As shown in in FIG. 12, the rectangular housing of vibratory stimulus generator element 336 may define a connection interface that is structurally and electrically mountable on a pad of frame 332 to place element 336 in electrical communication with controller 334, allowing for transmission of data and/or power.

Thermal generator element 342 may communicate a thermal energy 32B to the brain through nerves associated with the physiologic tissue (e.g., skin 2). As shown in FIGs. 11 and 12, thermal generator element 342 may be a counterpart to thermal generator elements 42, 142, and/or 242 and thus able to convert electricity into an amount of thermal energy recognizable as hot or cold by temperature receptors of skin 2, such the Ruffini corpuscle. As above, thermal generator element 342 may comprise a flexible thermoelectric generator having a rectangular housing and a connection interface that is structurally and electrically mountable on a pad of frame 332 to place thermal generator element 342 in electrical communication with controller 334, allowing for transmission of data and/or power. As shown in FIGs. 11 and 12, thermal generator element 342 may output thermal energy 32B to the physiologic tissue (e.g., skin 2) in signal direction SD when a contact surface of thermal generator element 342 is maintained against the tissue. As shown in FIG. 15, thermal generator element 342 also may output thermal energy 32B to the physiologic tissue (e.g., skin 2) through self-supporting body 633.

As shown in FIG. 15, optical generator element 348 may communicate an optical energy 32E to the brain of user 1 through nerves associated with their eyes, such as the optical nerves. As shown in FIGs. 11 and 12, optical generator element 348 may comprise a light emitting diode, such as a RBG diode operable with controller 334 to output wide spectrum of different colors and vary the outputs of color over time responsive to control signal 82 to a visual portion of energy signal 90. As shown in FIG. 12, optical generator element 348 may comprise a connection interface that is structurally and electrically mountable on a pad of frame 332 to place optical generator element 348 in electrical communication with controller 334, allowing for transmission of data and/or power. As noted above, controller 334 may comprise heat transfer elements (e.g., fins) operable with optical generator element 348 move heat away from the RBG diode and into the interior of self-supporting body 633, which may act as a heat sink.

As shown in FIG. 15, sensor 355 may measure and output physiological data about user 1, including any brain sensors and/or body sensors adapted to measure energies output directly to and/or indirectly from user 1. For example, sensor 355 may be a counterpart of sensor 255 and thus similarly may comprise any combination of known sensing technologies, such as: a heart sensor (e.g., PPG + Pulse Oximtery) adapted to output heart signals for user 1; a body sensor (e.g., IMU) adapted to output motion signals for user 1; and a breath sensor (e.g., PPG + Gyroscope) adapted to output breath signals for user 1. Sensor 355 may similarly compromise any sensing technology capable of generating measurement data for user 1 based on their body's electrical activity. As shown in FIG. 15, sensor may be positioned adjacent support surface 635 of self-supporting body 633 and configured to measure the physiological data when body 633 is grasped and/or when support surface 635 is positioned on the physiologic tissue (e.g., skin 2).

As shown in FIG. 15, sensor 355 may serve as a data source for data communication apparatus 600. For example, sensor 355 may be operable with controller 334 to output sensory data to processing unit 60, which may be operable with lines of code to responsively generate and/ or output a control signal 82 causing one or more of generator elements 336, 342, and/or 348 to communicate with their respective portions of energy signal 90. Because of the respective communications between housing 333 and self-supporting body 633, and between sensor 355 and processing unit 60, data communication apparatus 600 may be described as a data reactive device, in which control signal 82 may cause generator elements 336, 342, and/or 348 to output their respective energies 32A, 32B, and/or 32E to the physiologic tissue (e.g., skin 2) and/or self-supporting body 633 responsive to either input data 80 from a data source 81 (e.g., FIG. 5) or measurement data from sensor 355. In keeping with above, different energies 32 may be absorbed and/or amplified by body 633 causing it to vibrate with vibratory stimulus generator element 336, change temperature with thermal generator element 342, and/or be illuminated with optical generator element 348, making self-supporting body 533 an amplifier for energy generator 331.

Aspects of self-supporting body 633 may be modified to enhance is decorative value and change how it interacts with energies 32. For example, self-supporting body 633 may comprise a translucent or semi-opaque polymeric material that has been molded around housing 333 to fuse it with self-supporting body 633. As shown in FIG. 15, self-supporting body 633 also may comprise a plurality of additives 634 that are mixed into the translucent or semi-opaque polymeric material and suspended about energy generator 331 to modify a visual appearance of data communication apparatus 500. Additives 634 may comprise flecks of glass, metal, and/or any other material operable to create swirls and irregular patterns in self-supporting body 633 that may be illuminated, glow, and/or change color when exposed to optical energy 32E, allowing body 633 to mimic the appearance and aura of different types of crystals. As shown in FIG. 15, additives 634 also may comprise any thermally conductive material (e.g., metal plates and/or powders) operable to help distribute thermal energy 32B.

## Claims

1. An apparatus comprising:
a housing (33, 133, 233, 333) comprising:
an energy generator (31, 131, 231, 331, 431, 531) comprising a plurality of generator elements operable to output a plurality of different energy types (32) in a signal direction (SD) toward a physiologic tissue (2);
a frame (132, 232, 332) comprising a printed circuit board that mechanically supports and electrically connects the plurality of generator elements to each other and is operable to position the plurality of generator elements in the housing; and
a controller (34, 134, 234, 334) operable to distribute electricity to the plurality of generator elements;
each generator element of the plurality of generator elements being independently operable with the controller, when the energy generator with the housing is positioned relative to the physiologic tissue, to communicate with different nerves associated with the physiologic tissue by outputting a different portion of an energy signal (90) in the signal direction toward the physiologic tissue with one energy type of the plurality of different energy types,
the plurality of generator elements comprising:
an impact or vibratory stimulus generator element (36, 136, 236, 336) comprising a mechanical actuator that is operable to output an impact or vibratory energy (32A) of the plurality of different energy types by converting the electricity into a mechanical stimulus;
a thermal generator element (42, 142, 242, 342) comprising a thermoelectric generator that is operable to output a thermal energy (32B) of the plurality of different energy types by converting the electricity into a hot or a cold stimulus; and
a continuous air gap and thermal break between side surfaces of the impact or vibratory stimulus generator element and side surfaces of the thermal generator element.

2. The apparatus of claim 1, wherein the vibratory stimulus generator element comprises a coin vibration motor.

3. The apparatus of claim 1, wherein the thermoelectric generator (42, 142) comprises an open shape with a central opening and the vibratory generator element (36, 136) is located in the central opening, optionally wherein:
the open shape of the thermoelectric generator (42, 142) comprises an annular shape;
the central opening of the thermoelectric generator comprises a circular central opening; and
the vibratory generator element (36, 136) comprises a circular shape located in the circular central opening of the thermoelectric generator to define the continuous air gap and thermal break between the exterior surfaces of the vibratory generator element and the interior surfaces of the thermoelectric generator element.

4. The apparatus of claim 1, wherein the plurality of generator elements comprise an electrical stimulus generator element (48, 148, 248), optionally wherein the plurality of different energy types (32) comprise an electrical stimulus (32C) output with the electrical stimulus generator element.

5. The apparatus of claim 4, wherein the electrical stimulus generator element comprises a pair of contact plates (149, 249) operable to output the electrical stimulus, optionally wherein the pair of contact plates are structurally connected to the printed circuit board (132) by an insulating material and electrical connected to the printed circuit board by a conductor, optionally wherein the pair of contact plates are mounted directly to pads of the printed circuit board, further optionally wherein the electrical stimulus generator comprises a pair of semi-annular contact plates that are positioned in the continuous air gap and to maintain the thermal break.

6. The apparatus of claim 1, wherein the plurality of generator elements comprise a pressure generator element (52, 152, 252, 352) comprising an electroacoustic transducer that is operable to output a pressure energy (32D) by converting the electricity into a sound wave, wherein the plurality of different energy types comprise the pressure energy output with the pressure generator element, optionally wherein
the pressure generator element comprises one of:
a piezoelectric speaker (153) operable to output the pressure energy responsive to an electric current supplied to the pressure generator element, and, optionally wherein:
a face of the piezoelectric speaker is spaced apart from the physiologic tissue to define an air gap between the face and the physiologic tissue; and
the pressure energy comprises a sonic energy output toward the physiologic tissue through the air gap with the piezoelectric speaker;
an array of piezoelectric speakers (153) operable to output the pressure energy responsive to an electric current supplied to the array and, optionally, wherein the array of piezoelectric speakers comprises a radial array of piezoelectric speakers; and
an ultrasound transducer (153) operable to output an ultrasonic portion of the pressure energy toward the physiologic tissue.

7. The apparatus of claim 1, wherein the plurality of generator elements comprise an optical generator element (348) operable to output an optical energy, optionally wherein the plurality of different energy types (32) comprise the optical energy output with the optical generator element and, optionally, wherein:
the optical generator element comprises a multi-color LED that is operable to output the optical energy, and mechanically supported by and electrically connected to the printed circuit board.

8. The apparatus of claim 1, comprising a sensor (255, 355) that is operable to detect physiological signals associated with the physiologic tissue and mechanically supported by and electrically connected to the printed circuit board, optionally wherein:
the sensor is operable to detect physiological signals comprising measurements of electrical activity produced by a beating heart; or
wherein the sensor is operable to detect physiological signals comprising measurements of electrical activity produced by movements; or
wherein the sensor is operable to detect physiological signals comprising measurements of electrical activity produced by breathing.

9. The apparatus of claim 1, wherein the housing (33, 133, 233, 333) is embedded in a graspable body (433), optionally wherein the graspable body (433) comprises a cylindrical shape made from an insulating material operable to limit outputs of the plurality of different energy types (32) in directions away from the physiologic tissue (2).

10. The apparatus of claim 1, wherein the housing (33, 133, 233, 333) is embedded in a wearable body (533) operable to maintain a position of the plurality of energy generator elements relative to the physiologic tissue (2) when the wearable body is worn, optionally wherein the wearable body (533) is configured to absorb a portion of the plurality of energies (32) when the energy signal is output, optionally wherein the wearable body (533) comprises a crystalline structure or a polymeric structure, optionally wherein the wearable body (533) comprises an upper portion with an opening (534) sized to receive a cord or chain adapted to support the wearable body around a neck of a user so that a skin-facing surface (535) of the wearable body may be maintained against an area of skin on the chest of the user by gravity forces.

11. The apparatus of claim 1, wherein the housing is embedded in a support body (633) operable to maintain a position of the plurality of energy generator elements relative to the physiologic tissue (2) when the support body (633) is resting on the tissue.

12. The apparatus of claim 11, wherein the support body (633) comprises a crystalline structure or a polymeric structure; or
wherein one generator element of the plurality of generator elements is oriented toward an interior portion of the support body so that one portion of the energy signal (32) is output to the physiologic tissue (2) through the support body (633).

13. The apparatus of claim 11, wherein a portion of the impact or vibratory energy (32A) is transferred to the support body (633) when the vibratory generator element (36, 136, 236, 336) is activated; and a portion of the thermal energy (32B) is transferred to the support body (633) when the thermal generator (42, 142, 242, 342) element is activated.

14. The apparatus of claim 13, wherein the support body comprises a translucent polymeric structure and additives (634) that are suspended in the translucent polymeric structure and responsive to at least one energy type of the plurality of different energy types (32).

15. The apparatus of claim 14, wherein:
the plurality of generator elements comprise an optical generator element (348);
the plurality of different energy types (32) comprise an optical energy (32E) output with the optical generator element; and
the optical generator element comprises a multi-color LED operable to illuminate the translucent polymeric structure in a plurality of different colors with the optical energy, and, optionally:
wherein the additives comprise flecks of a light reactive material suspended in the translucent polymeric structure; or
wherein the additives comprise a thermally reactive material that is suspended in the translucent polymeric structure to define thermally conductive pathways.

## Patentansprüche

1. Einrichtung, umfassend:
ein Gehäuse (33, 133, 233, 333), das Folgendes umfasst:
einen Energiegenerator (31, 131, 231, 331, 431, 531), der eine Vielzahl von Generatorelementen umfasst, die betreibbar sind, um eine Vielzahl von verschiedenen Energiearten (32) in einer Signalrichtung (SD) zu einem physiologischen Gewebe (2) hin auszugeben;
einen Rahmen (132, 232, 332), der eine gedruckte Leiterplatte umfasst, die die Vielzahl von Generatorelementen mechanisch stützt und elektrisch miteinander verbindet und dazu betreibbar ist, die Vielzahl von Generatorelementen in dem Gehäuse zu positionieren; und
eine Steuerung (34, 134, 234, 334), die betreibbar ist, um Elektrizität an die Vielzahl von Generatorelementen zu verteilen;
wobei jedes Generatorelement der Vielzahl von Generatorelementen unabhängig mit der Steuerung betreibbar ist, wenn der Energiegenerator mit dem Gehäuse relativ zu dem physiologischen Gewebe positioniert ist, um mit unterschiedlichen Nerven zu kommunizieren, die dem physiologischen Gewebe zugeordnet sind, indem ein unterschiedlicher Anteil eines Energiesignals (90) in der Signalrichtung zu dem physiologischen Gewebe hin mit einer Energieart der Vielzahl von unterschiedlichen Energiearten ausgegeben wird,
wobei die Vielzahl von Generatorelementen Folgendes umfasst:
ein Stoß- oder Schwingungsstimulusgeneratorelement (36, 136, 236, 336), das einen mechanischen Aktor umfasst, der betreibbar ist, um eine Stoß- oder Schwingungsenergie (32A) der Vielzahl von unterschiedlichen Energiearten durch Umwandeln der Elektrizität in einen mechanischen Stimulus auszugeben;
ein Thermogeneratorelement (42, 142, 242, 342), das einen thermoelektrischen Generator umfasst, der betreibbar ist, um eine thermische Energie (32B) der Vielzahl von unterschiedlichen Energiearten durch Umwandeln der Elektrizität in einen heißen oder einen kalten Stimulus auszugeben; und
einen durchgehenden Luftspalt und eine thermische Trennung zwischen Seitenoberflächen des Stoß- oder Schwingungsstimulusgeneratorelements und Seitenoberflächen des Thermogeneratorelements.

2. Einrichtung nach Anspruch 1, wobei das Schwingungsstimulusgeneratorelement einen Münz-Schwingungsmotor umfasst.

3. Einrichtung nach Anspruch 1, wobei der thermoelektrische Generator (42, 142) eine offene Form mit einer zentralen Öffnung umfasst und sich das Schwingungsgeneratorelement (36, 136) in der zentralen Öffnung befindet, optional wobei:
die offene Form des thermoelektrischen Generators (42, 142) eine ringförmige Form umfasst;
die zentrale Öffnung des thermoelektrischen Generators eine kreisförmige zentrale Öffnung umfasst; und
das Schwingungsgeneratorelement (36, 136) eine kreisförmige Form umfasst, die sich in der kreisförmigen zentralen Öffnung des thermoelektrischen Generators befindet, um den durchgehenden Luftspalt und die thermische Trennung zwischen den Außenoberflächen des Schwingungsgeneratorelements und den Innenoberflächen des thermoelektrischen Generatorelements zu definieren.

4. Einrichtung nach Anspruch 1, wobei die Vielzahl von Generatorelementen ein elektrisches Stimulusgeneratorelement (48, 148, 248) umfasst, wobei die Vielzahl von unterschiedlichen Energiearten (32) optional einen elektrischen Stimulus (32C) umfasst, der mit dem elektrischen Stimulusgeneratorelement ausgegeben wird.

5. Einrichtung nach Anspruch 4, wobei das elektrische Stimulusgeneratorelement ein Paar Kontaktplatten (149, 249) umfasst, die betreibbar sind, um den elektrischen Stimulus auszugeben, wobei das Paar Kontaktplatten optional durch ein isolierendes Material strukturell mit der gedruckten Leiterplatte (132) verbunden ist und durch einen Leiter elektrisch mit der gedruckten Leiterplatte verbunden ist, wobei das Paar Kontaktplatten optional direkt an Pads der gedruckten Leiterplatte montiert ist, wobei der elektrische Stimulusgenerator ferner optional ein Paar halbringförmige Kontaktplatten umfasst, die in dem durchgehenden Luftspalt positioniert sind und die thermische Trennung aufrechterhalten.

6. Einrichtung nach Anspruch 1, wobei die Vielzahl von Generatorelementen ein Druckgeneratorelement (52, 152, 252, 352) umfasst, das einen elektroakustischen Wandler umfasst, der betreibbar ist, um eine Druckenergie (32D) durch Umwandeln der Elektrizität in eine Schallwelle auszugeben, wobei die Vielzahl von unterschiedlichen Energiearten die Druckenergie umfasst, die mit dem Druckgeneratorelement ausgegeben wird, wobei das Druckgeneratorelement optional eines von Folgenden umfasst:
einen piezoelektrischen Lautsprecher (153), der betreibbar ist, um die Druckenergie als Reaktion auf einen dem Druckgeneratorelement zugeführten elektrischen Strom auszugeben, und optional wobei:
eine Stirnfläche des piezoelektrischen Lautsprechers von dem physiologischen Gewebe beabstandet ist, um einen Luftspalt zwischen der Stirnfläche und dem physiologischen Gewebe zu definieren; und
die Druckenergie eine Schallenergie umfasst, die zu dem physiologischen Gewebe hin durch den Luftspalt mit dem piezoelektrischen Lautsprecher ausgegeben wird;
ein Array von piezoelektrischen Lautsprechern (153), das betreibbar ist, um die Druckenergie als Reaktion auf einen elektrischen Strom auszugeben, der dem Array zugeführt wird, und optional, wobei das Array von piezoelektrischen Lautsprechern ein radiales Array von piezoelektrischen Lautsprechern umfasst; und
einen Ultraschallwandler (153), der betreibbar ist, um einen Ultraschallanteil der Druckenergie zu dem physiologischen Gewebe hin auszugeben.

7. Einrichtung nach Anspruch 1, wobei die Vielzahl von Generatorelementen ein optisches Generatorelement (348) umfasst, das betreibbar ist, um eine optische Energie auszugeben, wobei die Vielzahl von unterschiedlichen Energiearten (32) optional die optische Energie umfasst, die mit dem optischen Generatorelement ausgegeben wird, und wobei optional:
das optische Generatorelement eine mehrfarbige LED umfasst, die betreibbar ist, um die optische Energie auszugeben, und mechanisch durch die gedruckte Leiterplatte gestützt und elektrisch damit verbunden ist.

8. Einrichtung nach Anspruch 1, umfassend einen Sensor (255, 355), der betreibbar ist, um physiologische Signale zu erkennen, die dem physiologischen Gewebe zugeordnet sind, und mechanisch durch die gedruckte Leiterplatte gestützt und elektrisch damit verbunden ist, optional wobei:
der Sensor dazu betreibbar ist, physiologische Signale zu erkennen, die Messungen einer elektrischen Aktivität umfassen, die durch ein schlagendes Herz erzeugt wird; oder
wobei der Sensor dazu betreibbar ist, physiologische Signale zu erkennen, die Messungen einer elektrischen Aktivität umfassen, die durch Bewegungen erzeugt wird; oder
wobei der Sensor dazu betreibbar ist, physiologische Signale zu erkennen, die Messungen einer elektrischen Aktivität umfassen, die durch Atmen erzeugt wird.

9. Einrichtung nach Anspruch 1, wobei das Gehäuse (33, 133, 233, 333) in einen greifbaren Hauptteil (433) eingebettet ist, wobei der greifbare Hauptteil (433) optional eine zylindrische Form umfasst, die aus einem isolierenden Material hergestellt ist, das betreibbar ist, um Ausgaben der Vielzahl von unterschiedlichen Energiearten (32) in Richtungen weg von dem physiologischen Gewebe (2) zu begrenzen.

10. Einrichtung nach Anspruch 1, wobei das Gehäuse (33, 133, 233, 333) in einen am Körper tragbaren Hauptteil (533) eingebettet ist, der betreibbar ist, um eine Position der Vielzahl von Energiegeneratorelementen relativ zu dem physiologischen Gewebe (2) aufrechtzuerhalten, wenn der am Körper tragbare Hauptteil am Körper getragen wird, optional wobei der am Körper tragbare Hauptteil (533) dazu konfiguriert ist, einen Anteil der Vielzahl von Energien (32) zu absorbieren, wenn das Energiesignal ausgegeben wird, optional wobei der am Körper tragbare Hauptteil (533) eine kristalline Struktur oder eine polymere Struktur umfasst, optional wobei der am Körper tragbare Hauptteil (533) einen oberen Anteil mit einer Öffnung (534) umfasst, die so bemessen ist, dass sie eine Schnur oder Kette aufnimmt, die so angepasst ist, dass sie den am Körper tragbaren Hauptteil um einen Hals eines Benutzers stützt, sodass eine der Haut zugewandte Oberfläche (535) des am Körper tragbaren Hauptteils durch Schwerkraft gegen einen Hautbereich auf der Brust des Benutzers gehalten werden kann.

11. Einrichtung nach Anspruch 1, wobei das Gehäuse in einen Stützhauptteil (633) eingebettet ist, der betreibbar ist, um eine Position der Vielzahl von Energiegeneratorelementen relativ zu dem physiologischen Gewebe (2) aufrechtzuerhalten, wenn der Stützhauptteil (633) an dem Gewebe anliegt.

12. Einrichtung nach Anspruch 11, wobei der Stützhauptteil (633) eine kristalline Struktur oder eine polymere Struktur umfasst; oder
wobei ein Generatorelement der Vielzahl von Generatorelementen zu einem Innenanteil des Stützhauptteils hin ausgerichtet ist, sodass ein Anteil des Energiesignals (32) durch den Stützhauptteil (633) an das physiologische Gewebe (2) ausgegeben wird.

13. Einrichtung nach Anspruch 11, wobei ein Anteil der Stoß- oder Schwingungsenergie (32A) auf den Stützhauptteil (633) übertragen wird, wenn das Schwingungsgeneratorelement (36, 136, 236, 336) aktiviert ist; und ein Anteil der thermischen Energie (32B) auf den Stützhauptteil (633) übertragen wird, wenn das Thermogeneratorelement (42, 142, 242, 342) aktiviert ist.

14. Einrichtung nach Anspruch 13, wobei der Stützhauptteil eine lichtdurchlässige Polymerstruktur und Additive (634) umfasst, die in der lichtdurchlässigen Polymerstruktur suspendiert sind und auf mindestens eine Energieart der Vielzahl von unterschiedlichen Energiearten (32) reagieren.

15. Einrichtung nach Anspruch 14, wobei:
die Vielzahl von Generatorelementen ein optisches Generatorelement (348) umfasst;
die Vielzahl von unterschiedlichen Energiearten (32) eine optische Energie (32E) umfasst, die mit dem optischen Generatorelement ausgegeben wird; und
das optische Generatorelement eine mehrfarbige LED umfasst, die betreibbar ist, um die lichtdurchlässige Polymerstruktur in einer Vielzahl von unterschiedlichen Farben mit der optischen Energie zu beleuchten, und optional:
wobei die Additive Partikel aus einem lichtreaktiven Material umfassen, die in der lichtdurchlässigen Polymerstruktur suspendiert sind; oder
wobei die Additive ein thermoreaktives Material umfassen, das in der lichtdurchlässigen Polymerstruktur suspendiert ist, um thermisch leitfähige Bahnen zu definieren.

## Revendications

1. Appareil comprenant :
un boîtier (33, 133, 233, 333) comprenant :
un générateur d'énergie (31, 131, 231, 331, 431, 531) comprenant une pluralité d'éléments générateurs servant à délivrer en sortie une pluralité de types d'énergie (32) différents dans une direction de signal (SD) vers un tissu physiologique (2) ;
un cadre (132, 232, 332) comprenant une carte de circuit imprimé qui supporte mécaniquement et connecte électriquement la pluralité d'éléments générateurs les uns aux autres et sert à positionner la pluralité d'éléments générateurs dans le boîtier ; et
un dispositif de commande (34, 134, 234, 334) servant à distribuer de l'électricité à la pluralité d'éléments générateurs ;
chaque élément générateur de la pluralité d'éléments générateurs servant indépendamment avec le dispositif de commande, lorsque le générateur d'énergie avec le boîtier est positionné par rapport au tissu physiologique, à communiquer avec différents nerfs associés au tissu physiologique en délivrant en sortie une partie différente d'un signal d'énergie (90) dans la direction du signal vers le tissu physiologique avec un type d'énergie de la pluralité de types d'énergie différents, la pluralité d'éléments générateurs comprenant :
un élément générateur de stimulus d'impact ou vibratoire (36, 136, 236, 336) comprenant un actionneur mécanique qui sert à délivrer en sortie une énergie d'impact ou vibratoire (32A) de la pluralité de types d'énergie différents en convertissant l'électricité en un stimulus mécanique ;
un élément générateur thermique (42, 142, 242, 342) comprenant un générateur thermoélectrique qui sert à délivrer en sortie une énergie thermique (32B) de la pluralité de types d'énergie différents en convertissant l'électricité en un stimulus chaud ou froid ; et
un entrefer continu et une rupture thermique entre les surfaces latérales de l'élément générateur de stimulus vibratoire ou d'impact et les surfaces latérales de l'élément générateur thermique.

2. Appareil de la revendication 1, dans lequel l'élément générateur de stimulus vibratoire comprend un moteur de vibration discoïdal.

3. Appareil de la revendication 1, dans lequel le générateur thermoélectrique (42, 142) comprend une forme ouverte avec une ouverture centrale et l'élément générateur vibratoire (36, 136) est situé dans l'ouverture centrale, éventuellement dans lequel :
la forme ouverte du générateur thermoélectrique (42, 142) comprend une forme annulaire ;
l'ouverture centrale du générateur thermoélectrique comprend une ouverture centrale circulaire ; et
l'élément générateur vibratoire (36, 136) comprend une forme circulaire située dans l'ouverture centrale circulaire du générateur thermoélectrique pour définir l'entrefer continu et la rupture thermique entre les surfaces extérieures de l'élément générateur vibratoire et les surfaces intérieures de l'élément générateur thermoélectrique.

4. Appareil de la revendication 1, dans lequel la pluralité d'éléments générateurs comprend un élément générateur de stimulus électrique (48, 148, 248), éventuellement dans lequel la pluralité de types d'énergie (32) différents comprend un stimulus électrique (32C) délivré en sortie avec l'élément générateur de stimulus électrique.

5. Appareil de la revendication 4, dans lequel l'élément générateur de stimulus électrique comprend une paire de plaques de contact (149, 249) servant à délivrer en sortie le stimulus électrique, éventuellement dans lequel la paire de plaques de contact est structurellement connectée à la carte de circuit imprimé (132) par un matériau isolant et électriquement connectée à la carte de circuit imprimé par un conducteur, éventuellement dans lequel la paire de plaques de contact est montée directement sur des plots de la carte de circuit imprimé, en outre éventuellement dans lequel le générateur de stimulus électrique comprend une paire de plaques de contact semi-annulaires qui sont positionnées dans l'entrefer continu et pour maintenir la rupture thermique.

6. Appareil de la revendication 1, dans lequel la pluralité d'éléments générateurs comprend un élément générateur de pression (52, 152, 252, 352) comprenant un transducteur électroacoustique qui sert à délivrer en sortie une énergie de pression (32D) en convertissant l'électricité en une onde sonore, dans lequel la pluralité de types d'énergie différents comprend l'énergie de pression délivrée en sortie avec l'élément générateur de pression, éventuellement dans lequel l'élément générateur de pression comprend l'un parmi :
un haut-parleur piézoélectrique (153) servant à délivrer en sortie l'énergie de pression en réponse à un courant électrique fourni à l'élément générateur de pression, et, éventuellement, dans lequel :
une face du haut-parleur piézoélectrique est espacée du tissu physiologique pour définir un entrefer entre la face et le tissu physiologique ; et
l'énergie de pression comprend une énergie sonique délivrée en sortie vers le tissu physiologique à travers l'entrefer avec le haut-parleur piézoélectrique ;
un réseau de haut-parleurs piézoélectriques (153) servant à délivrer en sortie l'énergie de pression en réponse à un courant électrique fourni au réseau et, éventuellement, dans lequel le réseau de haut-parleurs piézoélectriques comprend un réseau radial de haut-parleurs piézoélectriques ; et
un transducteur à ultrasons (153) servant à délivrer en sortie une partie ultrasonore de l'énergie de pression vers le tissu physiologique.

7. Appareil de la revendication 1, dans lequel la pluralité d'éléments générateurs comprend un élément générateur optique (348) servant à délivrer en sortie une énergie optique, éventuellement dans lequel la pluralité de types d'énergie (32) différents comprend l'énergie optique délivrée en sortie avec l'élément générateur optique et, éventuellement, dans lequel :
l'élément générateur optique comprend une DEL multicolore qui sert à délivrer en sortie l'énergie optique, et supportée mécaniquement par la carte de circuit imprimé et électriquement connectée à celle-ci.

8. Appareil de la revendication 1, comprenant un capteur (255, 355) qui sert à détecter des signaux physiologiques associés au tissu physiologique et supporté mécaniquement par la carte de circuit imprimé et électriquement connecté à celle-ci, éventuellement dans lequel :
le capteur sert à détecter des signaux physiologiques comprenant des mesures de l'activité électrique produite par un cœur qui bat ; ou
dans lequel le capteur sert à détecter des signaux physiologiques comprenant des mesures de l'activité électrique produite par des mouvements ; ou
dans lequel le capteur sert à détecter des signaux physiologiques comprenant des mesures de l'activité électrique produite par une respiration.

9. Appareil de la revendication 1, dans lequel le boîtier (33, 133, 233, 333) est encastré dans un corps saisissable (433), éventuellement dans lequel le corps saisissable (433) comprend une forme cylindrique faite d'un matériau isolant servant à limiter les sorties de la pluralité de types d'énergie (32) différents dans des directions s'éloignant du tissu physiologique (2).

10. Appareil de la revendication 1, dans lequel le boîtier (33, 133, 233, 333) est encastré dans un corps portable (533) servant à maintenir une position de la pluralité d'éléments générateurs d'énergie par rapport au tissu physiologique (2) lorsque le corps portable est porté, éventuellement dans lequel le corps portable (533) est conçu pour absorber une partie de la pluralité d'énergies (32) lorsque le signal d'énergie est délivré en sortie, éventuellement dans lequel le corps portable (533) comprend une structure cristalline ou une structure polymère, éventuellement dans lequel le corps portable (533) comprend une partie supérieure avec une ouverture (534) dimensionnée pour recevoir un cordon ou une chaîne adaptée pour supporter le corps portable autour d'un cou d'un utilisateur afin qu'une surface faisant face à la peau (535) du corps portable puisse être maintenue contre une zone de peau sur la poitrine de l'utilisateur par des forces de gravité.

11. Appareil de la revendication 1, dans lequel le boîtier est encastré dans un corps de support (633) servant à maintenir une position de la pluralité d'éléments générateurs d'énergie par rapport au tissu physiologique (2) lorsque le corps de support (633) repose sur le tissu.

12. Appareil de la revendication 11, dans lequel le corps de support (633) comprend une structure cristalline ou une structure polymère ; ou
dans lequel un élément générateur de la pluralité d'éléments générateurs est orienté vers une partie intérieure du corps de support afin qu'une partie du signal d'énergie (32) soit délivrée en sortie au tissu physiologique (2) à travers le corps de support (633).

13. Appareil de la revendication 11, dans lequel une partie de l'énergie d'impact ou vibratoire (32A) est transférée au corps de support (633) lorsque l'élément générateur vibratoire (36, 136, 236, 336) est activé ; et une partie de l'énergie thermique (32B) est transférée au corps de support (633) lorsque l'élément générateur thermique (42, 142, 242, 342) est activé.

14. Appareil de la revendication 13, dans lequel le corps de support comprend une structure polymère translucide et des additifs (634) qui sont en suspension dans la structure polymère translucide et sensibles à au moins un type d'énergie de la pluralité de types d'énergie (32) différents.

15. Appareil de la revendication 14, dans lequel :
la pluralité d'éléments générateurs comprend un élément générateur optique (348) ;
la pluralité de types d'énergie (32) différents comprend une énergie optique (32E) délivrée en sortie avec l'élément générateur optique ; et
l'élément générateur optique comprend une DEL multicolore servant à éclairer la structure polymère translucide dans une pluralité de couleurs différentes avec l'énergie optique, et, éventuellement :
dans lequel les additifs comprennent des flocons d'un matériau réactif léger en suspension dans la structure polymère translucide ; ou
dans lequel les additifs comprennent un matériau thermiquement réactif qui est en suspension dans la structure polymère translucide pour définir des voies thermiquement conductrices.
